# FASCICULE DE BREVET EUROPEEN

(11) **EP 1 346 230 B1**
(45) Date de publication et mention de la délivrance du brevet: **12.04.2006**
(21) Numéro de dépôt: 01995786.9
(22) Date de dépôt: 24.12.2001
(51) Int. Cl.: G01N 33/68, C07K 14/705, C07K 14/47, G01N 33/574

(54) **PROCEDE DE CRIBLAGE BASE SUR LES PARTENAIRES DE LIAISON DE TSAP6**
AUF TSAP6-BINDUNGSPARTNERN BERUHENDES SCREENINGVERFAHREN
SCREENING METHOD BASED ON TSAP6 BINDING PARTNERS

(30) Priorité: 26.12.2000 FR 0017027; 18.09.2001 WO PCT/FR01/02896
(43) Date de publication de la demande: 24.09.2003
(73) Titulaire: Cerenis, 75011 Paris (FR)
(72) Inventeur: AMSON, Robert, F-75005 Paris (FR); TELERMAN, Adam, F-75007 Paris (FR); PASSER, Brent, F-75005 Paris (FR)
(74) Mandataire: Warcoin, Jacques
(86) Numéro de dépôt international: PCT/FR2001/004188
(87) Numéro de publication internationale: WO 2002/052274

(56) Documents cités:
- WO-A-96/17061
- WO-A-97/22695
- US-A- 5 665 562
- AMSON R B ET AL: "ISOLATION OF 10 DIFFERENTIALLY EXPRESSED CNDAS IN P53-INDUCED APOPTOSIS: ACTIVATION OF THE VERTEBRATE HOMOLOGUE OF THE DROSOPHILA SEVEN IN ABSENTIA GENE" PROCEEDINGS OF THE NATIONAL ACADEMY OF SCIENCES OF USA, NATIONAL ACADEMY OF SCIENCE. WASHINGTON, US, vol. 93, no. 9, 30 avril 1996 (1996-04-30), pages 3953-3957, XP002032914 ISSN: 0027-8424
- STEINER MITCHELL S ET AL: "Growth inhibition of prostate cancer by an adenovirus expressing a novel tumor suppressor gene, pHyde." CANCER RESEARCH, vol. 60, no. 16, 15 août 2000 (2000-08-15), pages 4419-4425, XP001076638 ISSN: 0008-5472 -& DATABASE SWISS PROT [en ligne] 1 juin 2001 (2001-06-01) STEINER M S ET AL: "TUMOR SUPPRESSOR PHYDE" Database accession no. Q99P41 XP002198625
- DATABASE SWISS PROT [en ligne] 1 octobre 2000 (2000-10-01) SERRU V ET AL: "Dudulin 2, a new tumor antigen expressed in various human tumors" Database accession no. Q9NVB5 XP002198626
- BIOTECHNIQUES, vol. 7, no. 10, 1989, pages 1132-1138,
- ANALYTICAL BIOCHEMISTRY, vol. 243, 1996, pages 282-283,
- JOURNAL OF BIOMOLECULAR SCREENING, vol. 3, no. 2, 1998, pages 91-99,
- DDT, vol. 2, no. 9, 1997, pages 373-381,
- CELL BIOLOGY, vol. 9, février 1999 (1999-02), pages 57-60,
- PNAS, vol. 96, janvier 1999 (1999-01), pages 151-156,
- JOURNAL OF BIOMOLECULAR SCREENING, vol. 4, no. 6, 1999, pages 335-353,
- METHODS IN ENZYMOLOGY, vol. 246, 1995, pages 283-301,
- PNAS, vol. 91, décembre 1994 (1994-12), pages 12980-12984,
- CURRENT OPINION IN BIOTECHNOLOGY, vol. 8, 1997, pages 50-57,
- PNAS, vol. 94, août 1997 (1997-08), pages 8405-8410,
- CURRENT PROTOCOLS IN MOLECULAR BIOLOGY, 1998, page 10.17.1,
- CURRENT PROTOCOLS IN MOLECULAR BIOLOGY, 1999, page 10.16.1,
- JOURNAL OF IMMUNOLOGICAL METHODS, vol. 218, 1998, pages 1-8,

### LISTE DE SEQUENCES

<110> MOLECULAR ENGINES LABORATORIES - MEL
<120> PROCEDE DE CRIBLAGE BASE SUR LES PARTENAIRES DE LIAISON DE TSAP6
<130> D19307
<150> FR 00 17 027
   <151> 2000-12-26
<150> PCT/FR01/02896
   <151> 2001-09-18
<160> 52
<170> PatentIn Ver. 2.1
<210> 1
   <211> 218
   <212> PRT
   <213> Mus musculus
<220>
   <223> Mouse Nix-AF067395
<400> 1
<210> 2
   <211> 219
   <212> PRT
   <213> Homo sapiens
<220>
   <223> Human Nix-4138826
<400> 2
   Met Ser Ser His Leu Val Glu Pro Pro Pro Pro Leu His Asn Asn Asn
   1               5                   10                  15
   Asn Asn Cys Glu Glu Asn Glu Gln Ser Leu Pro Pro Pro Ala Gly Leu
               20                  25                  30
   Asn Ser Ser Trp Val Glu Leu Pro Met Asn Ser Ser Asn Gly Asn Asp
           35                  40                  45
   Asn Gly Asn Gly Lys Asn Gly Gly Leu Glu His Val Pro Ser Ser Ser
       50                  55                  60
   Ser Ile His Asn Gly Asp Met Glu Lys Ile Leu Leu Asp Ala Gln His
   65                  70                  75                  80
   Glu Ser Gly Gln Ser Ser Ser Arg Gly Ser Ser His Cys Asp Ser Pro
                   85                  90                  95
   Ser Pro Gln Glu Asp Gly Gln Ile Met Phe Asp Val Glu Met His Thr
               100                 105                 110
   Ser Arg Asp His Ser Ser Gln Ser Glu Glu Glu Val Val Glu Gly Glu
           115                 120                 125
   Lys Glu Val Glu Ala Leu Lys Lys Ser Ala Asp Trp Val Ser Asp Trp
       130                 135                 140
   Ser Ser Arg Pro Glu Asn Ile Pro Pro Lys Glu Phe His Phe Arg His
   145                 150                 155                 160
   Pro Lys Arg Ser Val Ser Leu Ser Met Arg Lys Ser Gly Ala Met Lys
                   165                 170                 175
   Lys Gly Gly Ile Phe Ser Ala Glu Phe Leu Lys Val Phe Ile Pro Ser
               180                 185                 190
   Leu Phe Leu Ser His Val Leu Ala Leu Gly Leu Gly Ile Tyr Ile Gly
           195                 200                 205
   Lys Arg Leu Ser Thr Pro Ser Ala Ser Thr Tyr
       210                 215
<210> 3
   <211> 206
   <212> PRT
   <213> Mus musculus
<220>
   <223> Mouse Nix y2H
<400> 3
   Asn Asn Asn Asn Asn Cys Glu Glu Gly Glu Gln Pro Leu Pro Pro Pro
   1               5                   10                  15
   Ala Gly Leu Asn Ser Ser Trp Val Glu Leu Pro Met Asn Ser Ser Asn
               20                  25                  30
   Gly Asn Glu Asn Gly Asn Gly Lys Asn Gly Gly Leu Glu His Val Pro
           35                  40                  45
   Ser Ser Ser Ser Ile His Asn Gly Asp Met Glu Lys Ile Leu Leu Asp
       50                  55                  60
   Ala Gln His Glu Ser Gly Gln Ser Ser Ser Arg Gly Ser Ser His Cys
   65                  70                  75                      80
   Asp Ser Pro Ser Pro Gln Glu Asp Gly Gln Ile Met Phe Asp Val Glu
                   85                  90                  95
   Met His Thr Ser Arg Asp His Ser Ser Gln Ser Glu Glu Glu Val Val
               100                 105                 110
   Glu Gly Glu Lys Glu Val Glu Ala Leu Lys Lys Ser Ala Asp Trp Val
           115                 120                 125
   Ser Asp Trp Ser Ser Arg Pro Glu Asn Ile Pro Pro Lys Glu Phe His
       130                 135                 140
   Phe Arg His Pro Lys Arg Ala Ala Ser Leu Ser Met Arg Lys Ser Gly
   145                 150                 155                 160
   Ala Met Lys Lys Gly Gly Ile Phe Ser Ala Glu Phe Leu Lys Val Phe
                   165                 170                 175
   Ile Pro Ser Leu Phe Leu Ser His Val Leu Ala Leu Gly Leu Gly Ile
               180                 185                 190
   Tyr Ile Gly Lys Arg Leu Ser Thr Pro Ser Ala Ser Thr Tyr
           195                 200                 205
<210> 4
   <211> 175
   <212> PRT
   <213> Mus musculus
<220>
   <223> Mouse Siva-AF033112
<400> 4
   Met Pro Lys Arg Ser Cys Pro Phe Ala Asp Ala Ala Pro Leu Gln Leu
   1 5 10 15
   Lys Val His Val Gly Leu Lys Glu Leu Ser His Gly Val Phe Ala Glu
   20 25 30
   Arg Tyr Ser Arg Glu Val Phe Glu Arg Thr Lys Gln Leu Leu Phe Gln
   35 40 45
   Gly Ala Arg Ala Tyr Arg Asp His Ile Ser Ser Glu Asp Cys Ser Val
   50 55 60
   Asn His Leu Gln Glu Ser Leu Lys Ser Gly Val Val Gly Ala Pro Gln
   65 70 75 80
   Pro Ala Arg Gly Gln Met Leu Ile Gly Pro Asp Gly Arg Leu Thr Arg
   85 90 95
   Cys Gln Ala Gln Ala Ser Glu Gly Gly Leu Pro Arg Thr Ala Pro Ile
   100 105 110
   Ala Cys Ser Ser Cys Met Arg Ser Val Asp Gly Lys Ala Val Cys Ser
   115 120 125
   Gln Cys Glu Arg Ala Leu Cys Gly Gln Cys Val Tyr Thr Ser Trp Gly
   130 135 140
   Cys Gly Ala Leu Ala Cys Val Leu Cys Gly Leu Ala Asp Tyr Ala Asp
   145 150 155 160
   Asp Gly Glu Lys Thr Leu Cys Thr Ser Cys Ala Met Phe Glu Ala
   165 170 175
<210> 5
   <211> 124
   <212> PRT
   <213> Homo sapiens
<220>
   <223> Human Siva-HSU82938
<400> 5
<210> 6
   <211> 197
   <212> PRT
   <213> Mus musculus
<220>
   <223> Mouse Siva y2H
<400> 6
   Phe Gly Ser Glu Ala Lys Asn Ser Ala Arg Gly Leu Gly Ala Arg Arg
   1 5 10 15
   Ala Ala Arg Pro Ala Thr Met Pro Lys Arg Ser Cys Pro Phe Ala Asp
   20 25 30
   Ala Ala Pro Leu Gln Leu Lys Val His Val Gly Leu Lys Glu Leu Ser
   35 40 45
   His Gly Val Phe Ala Glu Arg Tyr Ser Arg Glu Val Phe Glu Arg Thr
   50 55 60
   Lys Gln Leu Leu Phe Gln Gly Ala Arg Ala Tyr Arg Asp His Ile Ser
   65 70 75 80
   Ser Glu Asp Cys Ser Val Asn His Leu Gln Glu Ser Leu Lys Ser Gly
   85 90 95
   Val Val Gly Ala Pro Gln Pro Ala Arg Gly Gln Met Leu Ile Gly Pro
   100 105 110
   Asp Gly Arg Leu Thr Arg Cys Gln Ala Gln Ala Ser Glu Gly Gly Leu
   115 120 125
   Pro Arg Thr Ala Pro Ile Ala Cys Ser Ser Cys Met Arg Ser Val Asp
   130 135 140
   Gly Lys Ala Val Cys Ser Gln Cys Glu Arg Ala Leu Cys Gly Gln Cys
   145 150 155 160
   Val Tyr Thr Ser Trp Gly Cys Gly Ala Leu Ala Cys Val Leu Cys Gly
   165 170 175
   Leu Ala Asp Tyr Ala Asp Asp Gly Glu Lys Thr Leu Cys Thr Ser Cys
   180 185 190
   Ala Met Phe Glu Ala
   195
<210> 7
   <211> 490
   <212> PRT
   <213> Mus musculus
<220>
   <223> Mouse Myt1 kinase-AF175892
<400> 7
   Met Thr Met Pro Thr Glu Gly Thr Pro Pro Pro Leu Ser Gly Thr Pro
   1 5 10 15
   Ile Pro Val Pro Ala Tyr Phe Arg His Ala Glu Pro Gly Phe Ser Leu
   20 25 30
   Lys Arg Pro Gly Gly Leu Ser Arg Ser Leu Pro Pro Arg Pro Pro Ala
           35                  40                  45
   Lys Gly Cys Ile Pro Val Ser Arg Leu Phe Pro Pro Arg Thr Pro Gly
       50                  55                  60
   Trp His Gln Pro Gln Pro Arg Arg Val Ser Phe Leu Cys Glu Thr Ser
   65                  70                  75                  80
   Glu Pro Leu Gln Ser Pro Gly Tyr Asp Pro Ser Arg Pro Glu Ser Phe
                   85                  90                  95
   Phe Gln Gln Asn Phe Gln Arg Leu Ser Arg Leu Gly His Gly Ser Tyr
               100                 105                 110
   Gly Glu Val Phe Lys Val Arg Ser Lys Glu Asp Gly Arg Leu Tyr Ala
           115                 120                 125
   Val Lys Arg Tyr Met Ser Pro Phe Arg Gly Pro Lys Asp Arg Thr Arg
       130                 135                 140
   Lys Leu Ala Glu Val Gly Gly His Glu Lys Val Gly Gln His Pro His
   145                 150                 155                 160
   Cys Val Arg Leu Glu Arg Ala Trp Glu Glu Gly Gly Ile Leu Tyr Leu
                   165                 170                 175
   Gln Thr Glu Leu Cys Gly Pro Ser Leu Gln Gln His Cys Glu Ala Trp
               180                 185                 190
   Gly Ala Ser Leu Pro Glu Ala Gln Val Trp Gly Tyr Leu Arg Asp Ile
           195                 200                 205
   Leu Leu Ala Leu Asp His Leu His Ser Gln Gly Leu Val His Leu Asp
       210                 215                 220
   Val Lys Pro Ala Asn Ile Phe Leu Gly Pro Arg Gly Arg Cys Lys Leu
   225                 230                 235                 240
   Gly Asp Phe Gly Leu Leu Val Glu Leu Gly Ser Thr Gly Ala Gly Glu
                   245                 250                 255
   Ala Gln Glu Gly Asp Pro Arg Tyr Met Ala Pro Glu Leu Leu Gln Gly
               260                 265                 270
   Ser Tyr Gly Thr Ala Ala Asp Val Phe Ser Leu Gly Leu Thr Ile Leu
           275                 280                 285
   Glu Val Ala Cys Asn Met Glu Leu Pro His Gly Gly Glu Gly Trp Gln
       290                 295                 300
   Gln Leu Arg Gln Gly Tyr Leu Pro Pro Glu Phe Thr Ala Gly Leu Ser
   305                 310                 315                 320
   Ser Glu Leu Arg Ser Val Leu Ala Met Met Leu Glu Pro Asp Pro Gln
                   325                 330                 335
   Leu Arg Ala Thr Ala Glu Ala Leu Leu Ala Leu Pro Met Leu Arg Gln
               340                 345                 350
   Pro Arg Pro Trp Asn Val Leu Trp Tyr Met Ala Ala Glu Ala Leu Ser
               355                     360                     365
   Arg Gly Trp Ala Leu Trp Gln Ala Leu Val Thr Leu Leu Cys Trp Leu
       370                     375                     380
   Trp His Gly Leu Val His Pro Ala Ser Trp Leu Gln Pro Pro Gly Pro
   385                     390                     395                     400
   Pro Ala Thr Pro Leu Gly Ser Pro Pro Cys Ser Pro Leu Met Asn Ser
                   405                     410                     415
   Thr Leu Ser Ser Ser Trp Asp Asn Asp Ser Ile Gly Pro Ser Leu Ser
               420                     425                     430
   Pro Glu Thr Val Leu Ser Arg Ile Thr Arg Arg Thr Ser Thr Pro Arg
           435                     440                     445
   Gly Arg Tyr Ile Pro Arg Asp Ala Leu Asp Leu Thr Asp Val Asp Ser
       450                     455                     460
   Glu Pro Pro Arg Gly Pro Cys Pro Thr Phe Glu Pro Arg Asn Leu Leu
   465                     470                     475                     480
   Ser Leu Phe Glu Asp Ser Leu Asp Pro Ala
                   485                     490
<210> 8
   <211> 499
   <212> PRT
   <213> Homo sapiens
<220>
   <223> Human Mytl kinase-2914674
<400> 8
   Met Leu Glu Arg Pro Pro Ala Leu Ala Met Pro Met Pro Thr Glu Gly
    1               5                  10                  15
   Thr Pro Pro Pro Leu Ser Gly Thr Pro Ile Pro Val Pro Ala Tyr Phe
               20                  25                  30
   Arg His Ala Glu Pro Gly Phe Ser Leu Lys Arg Pro Arg Gly Leu Ser
           35                  40                  45
   Arg Ser Leu Pro Pro Pro Pro Pro Ala Lys Gly Ser Ile Pro Ile Ser
       50                  55                  60
   Arg Leu Phe Pro Pro Arg Thr Pro Gly Trp His Gln Leu Gln Pro Arg
   65                  70                  75                  80
   Arg Val Ser Phe Arg Gly Glu Ala Ser Glu Thr Leu Gln Ser Pro Gly
                   85                  90                  95
   Tyr Asp Pro Ser Arg Pro Glu Ser Phe Phe Gln Gln Ser Phe Gln Arg
               100                 105                 110
   Leu Ser Arg Leu Gly His Gly Ser Tyr Gly Glu Val Phe Lys Val Arg
           115                 120                 125
   Ser Lys Glu Asp Gly Arg Leu Tyr Ala Val Lys Arg Ser Met Ser Pro
   130 135 140
   Phe Arg Gly Pro Lys Asp Arg Ala Arg Lys Leu Ala Glu Val Gly Ser
   145 150 155 160
   His Glu Lys Val Gly Gln His Pro Cys Cys Val Arg Leu Glu Gln Ala
   165 170 175
   Trp Glu Glu Gly Gly Ile Leu Tyr Leu Gln Thr Glu Leu Cys Gly Pro
   180 185 190
   Ser Leu Gln Gln His Cys Glu Ala Trp Gly Ala Ser Leu Pro Glu Ala
   195 200 205
   Gln Val Trp Gly Tyr Leu Arg Asp Thr Leu Leu Ala Leu Ala His Leu
   210 215 220
   His Ser Gln Gly Leu Val His Leu Asp Val Lys Pro Ala Asn Ile Phe
   225 230 235 240
   Leu Gly Pro Arg Gly Arg Cys Lys Leu Gly Asp Phe Gly Leu Leu Val
   245 250 255
   Glu Leu Gly Thr Ala Gly Ala Gly Glu Val Gln Glu Gly Asp Pro Arg
   260 265 270
   Tyr Met Ala Pro Glu Leu Leu Gln Gly Ser Tyr Gly Thr Ala Ala Asp
   275 280 285
   Val Phe Ser Leu Gly Leu Thr Ile Leu Glu Val Ala Cys Asn Met Glu
   290 295 300
   Leu Pro His Gly Gly Glu Gly Trp Gln Gln Leu Arg Gln Gly Tyr Leu
   305 310 315 320
   Pro Pro Glu Phe Thr Ala Gly Leu Ser Ser Glu Leu Arg Ser Val Leu
   325 330 335
   Val Met Met Leu Glu Pro Asp Pro Lys Leu Arg Ala Thr Ala Glu Ala
   340 345 350
   Leu Leu Ala Leu Pro Val Leu Arg Gln Pro Arg Ala Trp Gly Val Leu
   355 360 365
   Trp Cys Met Ala Ala Glu Ala Leu Ser Arg Gly Trp Ala Leu Trp Gln
   370 375 380
   Ala Leu Leu Ala Leu Leu Cys Trp Leu Trp His Gly Leu Ala His Pro
   385 390 395 400
   Ala Ser Trp Leu Gln Pro Leu Gly Pro Pro Ala Thr Pro Pro Gly Ser
   405 410 415
   Pro Pro Cys Ser Leu Leu Leu Asp Ser Ser Leu Ser Ser Asn Trp Asp
   420 425 430
   Asp Asp Ser Leu Gly Pro Ser Leu Ser Pro Glu Ala Val Leu Ala Arg
   435 440 445
   Thr Val Gly Ser Thr Ser Thr Pro Arg Ser Arg Cys Thr Pro Arg Asp
       450                 455                 460
   Ala Leu Asp Leu Ser Asp Ile Asn Ser Glu Pro Pro Arg Gly Ser Phe
   465                 470                 475                 480
   Pro Ser Phe Glu Pro Arg Asn Leu Leu Ser Leu Phe Glu Asp Thr Leu
                   485                 490                 495
   Asp Pro Thr
<210> 9
   <211> 150
   <212> PRT
   <213> Mus musculus
<220>
   <223> Mouse kinase y2h
<400> 9
   Ala Glu Ala Leu Leu Ala Leu Pro Met Leu Arg Gln Pro Arg Pro Trp
   1               5                   10                  15
   Asn Val Leu Trp Tyr Met Ala Ala Glu Ala Leu Ser Arg Gly Trp Ala
               20                  25                  30
   Leu Trp Gln Ala Leu Val Thr Leu Leu Cys Trp Leu Trp His Gly Leu
           35                  40                  45
   Val His Pro Ala Ser Trp Leu Gln Pro Pro Gly Pro Pro Ala Thr Pro
       50                  55                  60
   Leu Gly Ser Pro Pro Cys Ser Pro Leu Met Asn Ser Thr Leu Ser Ser
   65                  70                  75                  80
   Ser Trp Asp Asn Asp Ser Ile Gly Pro Ser Leu Ser Pro Glu Thr Val
                   85                  90                  95
   Leu Ser Arg Ile Thr Arg Arg Thr Ser Thr Pro Arg Gly Arg Tyr Ile
               100                 105                 110
   Pro Arg Asp Ala Leu Asp Leu Thr Asp Val Asp Ser Glu Pro Pro Arg
           115                 120                 125
   Gly Pro Cys Pro Thr Phe Glu Pro Arg Asn Leu Leu Ser Leu Phe Glu
       130                 135                 140
   Asp Ser Leu Asp Pro Ala
   145                 150
<210> 10
   <211> 178
   <212> PRT
   <213> Mus musculus
<220>
   <223> Mouse Cyclin A2-X75483
<400> 10
   Met Pro Gly Thr Ser Arg His Ser Gly Arg Asp Ala Gly Ser Ala Leu
   1               5                   10                  15
   Leu Ser Leu His Gln Glu Asp Gln Glu Asn Val Asn Pro Glu Lys Leu
               20                  25                  30
   Ala Pro Ala Gln Gln Pro Arg Ala Gln Ala Val Leu Lys Ala Gly Asn
           35                  40                  45
   Val Arg Gly Pro Ala Pro Gln Gln Lys Leu Lys Thr Arg Arg Val Ala
       50                  55                  60
   Pro Leu Lys Asp Leu Pro Ile Asn Asp Glu His Val Thr Ala Gly Pro
   65                  70                  75                  80
   Ser Trp Lys Ala Val Ser Lys Gln Pro Ala Phe Thr Ile His Val Asp
                   85                  90                  95
   Glu Ala Glu Glu Thr Gln Lys Arg Pro Ala Glu Leu Lys Glu Thr Glu
               100                 105                 110
   Cys Glu Asp Ala Leu Ala Phe Asn Ala Ala Val Ser Leu Pro Ala Ala
           115                 120                 125
   Arg Lys Pro Leu Thr Pro Leu Asp Tyr Pro Met Asp Gly Ser Phe Glu
       130                 135                 140
   Ser Pro His Ala Met Asp Met Ser Ile Val Leu Glu Asp Lys Pro Val
   145                 150                 155                 160
   Asn Val Asn Glu Val Pro Asp Tyr Gln Glu Asp Ile His Thr Tyr Leu
                   165                 170                 175
   Arg Glu
<210> 11
   <211> 244
   <212> PRT
   <213> Homo sapiens
<220>
   <223> Human Cyclin A2-510604
<400> 11
   Met Glu Val Lys Cys Lys Pro Lys Val Gly Tyr Met Lys Arg Gln Pro
   1 5 10 15
   Asp Ile Thr Asn Ser Met Arg Ala Ile Leu Val Asp Trp Leu Val Glu
   20 25 30
   Val Gly Glu Glu Tyr Lys Leu Gln Asn Glu Thr Leu His Leu Ala Val
   35 40 45
   Asn Tyr Ile Asp Arg Phe Leu Ser Ser Met Ser Val Leu Arg Gly Lys
   50 55 60
   Leu Gln Leu Val Gly Thr Ala Ala Met Leu Leu Ala Ser Lys Phe Glu
   65 70 75 80
   Glu Ile Tyr Pro Pro Glu Val Ala Glu Phe Val Tyr Ile Thr Asp Asp
   85 90 95
   Thr Tyr Ser Lys Lys Gln Val Leu Arg Met Glu His Leu Val Leu Lys
   100 105 110
   Val Leu Ala Phe Asp Leu Ala Ala Pro Thr Val Asn Gln Phe Leu Thr
   115 120 125
   Gln Tyr Phe Leu His Leu Gln Pro Ala Asn Cys Lys Val Glu Ser Leu
   130 135 140
   Ala Met Phe Leu Gly Glu Leu Ser Leu Ile Asp Ala Asp Pro Tyr Leu
   145 150 155 160
   Lys Tyr Leu Pro Ser Leu Ile Ala Gly Ala Ala Phe His Leu Ala Leu
   165 170 175
   Tyr Thr Val Thr Gly Gln Ser Trp Pro Glu Ser Leu Ala Gln Gln Thr
   180 185 190
   Gly Tyr Thr Leu Glu Ser Leu Lys Pro Cys Leu Val Asp Leu His Gln
   195 200 205
   Thr Tyr Leu Lys Pro Pro Gln His Ala Gln Gln Ser Ile Arg Glu Lys
   210 215 220
   Tyr Lys His Ser Lys Tyr His Ser Val Ser Leu Leu Asn Pro Pro Glu
   225 230 235 240
   Thr Leu Ser Val
<210> 12
   <211> 75
   <212> PRT
   <213> Mus musculus
<220>
   <223> Mouse Cyclin A2 y2H
<400> 12
   Ala Ala Phe His Leu Ala Leu Tyr Thr Val Thr Gly Gln Ser Trp Pro
   1               5                   10                  15
   Glu Ser Leu Ala Gln Gln Thr Gly Tyr Thr Leu Glu Ser Leu Lys Pro
               20                  25                  30
   Cys Leu Val Asp Leu His Gln Thr Tyr Leu Lys Pro Pro Gln His Ala
           35                  40                  45
   Gln Gln Ser Ile Arg Glu Lys Tyr Lys His Ser Lys Tyr His Ser Val
       50                  55                  60
   Ser Leu Leu Asn Pro Pro Glu Thr Leu Ser Val
   65                  70                  75
<210> 13
   <211> 438
   <212> PRT
   <213> Mus musculus
<220>
   <223> Mouse PM-SCL-9506980
<400> 13
   Met Lys Glu Thr Pro Leu Ser Asn Cys Glu Arg Arg Phe Leu Leu Arg
   1               5                   10                  15
   Ala Ile Glu Glu Lys Lys Arg Leu Asp Gly Arg Gln Thr Tyr Asp Tyr
               20                  25                  30
   Arg Asn Ile Arg Ile Ser Phe Gly Thr Asp Tyr Gly Cys Cys Ile Val
           35                  40                  45
   Glu Leu Gly Lys Thr Arg Val Leu Gly Gln Val Ser Cys Glu Leu Val
       50                  55                  60
   Ser Pro Lys Leu Asn Arg Ala Thr Glu Gly Ile Leu Phe Phe Asn Leu
   65                  70                  75                  80
   Glu Leu Ser Gln Met Ala Ala Pro Ala Phe Glu Pro Gly Arg Gln Ser
                   85                  90                  95
   Asp Leu Leu Val Lys Leu Asn Arg Leu Leu Glu Arg Cys Leu Arg Asn
               100                 105                 110
   Ser Lys Cys Ile Asp Thr Glu Ser Leu Cys Val Val Ala Gly Glu Lys
           115                 120                 125
   Val Trp Gln Ile Arg Val Asp Leu His Leu Leu Asn His Asp Gly Asn
       130                 135                 140
   Ile Ile Asp Ala Ala Ser Ile Ala Ala Ile Val Ala Leu Cys His Phe
   145                 150                 155                 160
   Arg Arg Pro Asp Val Ser Val Gln Gly Glu Glu Val Thr Leu Tyr Thr
                   165                 170                 175
   Pro Glu Glu Arg Asp Pro Val Pro Leu Ser Ile His His Met Pro Ile
               180                 185                 190
   Cys Val Ser Phe Ala Phe Phe Gln Gln Gly Thr Tyr Leu Leu Val Asp
           195                 200                 205
   Pro Asn Glu Arg Glu Glu Arg Val Met Asp Gly Leu Leu Val Ile Ala
       210                 215                 220
   Met Asn Lys His Arg Glu Ile Cys Thr Ile Gln Ser Ser Gly Gly Ile
   225                 230                 235                 240
   Met Leu Leu Lys Asp Gln Val Phe Arg Cys Ser Lys Ile Ala Gly Val
                   245                 250                 255
   Lys Val Ala Glu Ile Thr Glu Leu Ile Gln Lys Ala Leu Glu Asn Asp
   260 265 270
   Gln Arg Val Arg Lys Glu Gly Gly Lys Phe Gly Phe Ala Glu Ser Ile
   275 280 285
   Ala Asn Gln Arg Ile Thr Ala Phe Lys Met Glu Thr Ala Pro Ile Asp
   290 295 300
   Thr Ser Asn Ile Glu Glu Arg Ala Glu Glu Ile Ile Ala Glu Ala Glu
   305 310 315 320
   Pro Pro Pro Glu Val Val Ser Gln Pro Val Leu Trp Thr Pro Gly Thr
   325 330 335
   Ala Gln Ile Gly Asp Gly Ile Glu Asn Ser Trp Gly Asp Leu Glu Asp
   340 345 350
   Ser Glu Lys Glu Glu Glu Glu Glu Glu Gly Gly Ile Asp Glu Ala Val
   355 360 365
   Ile Leu Asp Asp Thr Lys Met Asp Thr Gly Glu Val Ser Asp Ile Gly
   370 375 380
   Ser Gln Gly Ala Pro Ile Val Leu Ser Asp Ser Glu Glu Glu Glu Met
   385 390 395 400
   Ile Ile Leu Glu Pro Glu Lys Asn Pro Lys Lys Ile Arg Ala Gln Thr
   405 410 415
   Ser Ala Asn Gln Lys Ala Pro Ser Lys Gly Gln Gly Lys Arg Lys Lys
   420 425 430
   Lys Lys Arg Thr Ala Asn
   435
<210> 14
   <211> 104
   <212> PRT
   <213> Mus musculus
<220>
   <223> Mouse PM-SCL y2h
<400> 14
   Gly Thr Ala Gln Ile Gly Asp Gly Ile Glu Asn Ser Trp Gly Asp Leu
   1 5 10 15
   Glu Asp Ser Glu Lys Glu Glu Glu Glu Glu Glu Gly Gly Ile Asp Glu
   20 25 30
   Ala Val Ile Leu Asp Asp Thr Lys Met Asp Thr Gly Glu Val Ser Asp
   35 40 45
   Ile Gly Ser Gln Gly Ala Pro Ile Val Leu Ser Asp Ser Glu Glu Glu
   50 55 60
   Glu Met Ile Ile Leu Glu Pro Glu Lys Asn Pro Lys Lys Ile Arg Ala
   65 70 75 80
   Gln Thr Ser Ala Asn Gln Lys Ala Pro Ser Lys Gly Gln Gly Lys Arg
   85 90 95
   Lys Lys Lys Lys Arg Thr Ala Asn
   100
<210> 15
   <211> 255
   <212> PRT
   <213> Mus musculus
<220>
   <223> Mouse C8 preteaseme-NM_011181
<400> 15
   Met Ser Ser Ile Gly Thr Gly Tyr Asp Leu Ser Ala Ser Thr Phe Ser
   1               5                   10                  15
   Pro Asp Gly Arg Val Phe Gln Val Glu Tyr Ala Met Lys Ala Val Glu
               20                  25                  30
   Asn Ser Ser Thr Ala Ile Gly Ile Arg Cys Lys Asp Gly Val Val Phe
           35                  40                  45
   Gly Val Glu Lys Leu Val Leu Ser Lys Leu Tyr Glu Glu Gly Ser Asn
       50                  55                  60
   Lys Arg Leu Phe Asn Val Asp Arg His Val Gly Met Ala Val Ala Gly
   65                  70                  75                  80
   Leu Leu Ala Asp Ala Arg Ser Leu Ala Asp Ile Ala Arg Glu Glu Ala
                   85                  90                  95
   Ser Asn Phe Arg Ser Asn Phe Gly Tyr Asn Ile Pro Leu Lys His Leu
               100                 105                 110
   Ala Asp Arg Val Ala Met Tyr Val His Ala Tyr Thr Leu Tyr Ser Ala
           115                 120                 125
   Val Arg Pro Phe Gly Cys Ser Phe Met Leu Gly Ser Tyr Ser Ala Asn
       130                 135                 140
   Asp Gly Ala Gln Leu Tyr Met Ile Asp Pro Ser Gly Val Ser Tyr Gly
   145                 150                 155                 160
   Tyr Trp Gly Cys Ala Ile Gly Lys Ala Arg Gln Ala Ala Lys Thr Glu
                   165                 170                 175
   Ile Glu Lys Leu Gln Met Lys Glu Met Thr Cys Arg Asp Val Val Lys
               180                 185                 190
   Glu Val Ala Lys Ile Ile Tyr Ile Val His Asp Glu Val Lys Asp Lys
           195                 200                 205
   Ala Phe Glu Leu Glu Leu Ser Trp Val Gly Glu Leu Thr Lys Gly Arg
       210                 215                 220
   His Glu Ile Val Pro Lys Asp Ile Arg Glu Glu Ala Glu Lys Tyr Ala
   225                 230                 235                 240
   Lys Glu Ser Leu Lys Glu Glu Asp Glu Ser Asp Asp Asp Asn Met
                   245                 250                 255
<210> 16
   <211> 251
   <212> PRT
   <213> Mus musculus
<220>
   <223> Mouse C8 proteasome
<400> 16
   Gly Thr Gly Tyr Asp Leu Ser Ala Ser Thr Phe Ser Pro Asp Gly Arg
   1               5                   10                  15
   Val Phe Gln Val Glu Tyr Ala Met Lys Ala Val Glu Asn Ser Ser Thr
               20                  25                  30
   Ala Ile Gly Ile Arg Cys Lys Asp Gly Val Val Phe Gly Val Glu Lys
           35                  40                  45
   Leu Val Leu Ser Lys Leu Tyr Glu Glu Gly Ser Asn Lys Arg Leu Phe
       50                  55                  60
   Asn Val Asp Arg His Val Gly Met Ala Val Ala Gly Leu Leu Ala Asp
   65                  70                  75                  80
   Ala Arg Ser Leu Ala Asp Ile Ala Arg Glu Glu Ala Ser Asn Phe Arg
                   85                  90                  95
   Ser Asn Phe Gly Tyr Asn Ile Pro Leu Lys His Leu Ala Asp Arg Val
               100                 105                 110
   Ala Met Tyr Val His Ala Tyr Thr Leu Tyr Ser Ala Val Arg Pro Phe
           115                 120                 125
   Gly Cys Ser Phe Met Leu Gly Ser Tyr Ser Ala Asn Asp Gly Ala Gln
       130                 135                 140
   Leu Tyr Met Ile Asp Pro Ser Gly Val Ser Tyr Gly Tyr Trp Gly Cys
   145                 150                 155                 160
   Ala Ile Gly Lys Ala Arg Gln Ala Ala Lys Thr Glu Ile Glu Lys Leu
                   165                 170                 175
   Gln Met Lys Glu Met Thr Cys Arg Asp Val Val Lys Glu Val Ala Lys
               180                 185                 190
   Ile Ile Tyr Ile Val His Asp Glu Val Lys Asp Lys Ala Phe Glu Leu
           195                 200                 205
   Glu Leu Ser Trp Val Gly Glu Leu Thr Lys Gly Arg His Glu Ile Val
       210                 215                 220
   Pro Lys Asp Ile Arg Glu Glu Ala Glu Lys Tyr Ala Lys Glu Ser Leu
   225                 230                 235                 240
   Lys Glu Glu Asp Glu Ser Asp Asp Asp Asn Met
<210> 17
   <211> 1071
   <212> PRT
   <213> Homo sapiens
<220>
   <223> Human CRM1-2626839
<400> 17
   Met Pro Ala Ile Met Thr Met Leu Ala Asp His Ala Ala Arg Gln Leu
   1               5                   10                  15
   Leu Asp Phe Ser Gln Lys Leu Asp Ile Asn Leu Leu Asp Asn Val Val
               20                  25                  30
   Asn Cys Leu Tyr His Gly Glu Gly Ala Gln Gln Arg Met Ala Gln Glu
           35                  40                  45
   Val Leu Thr His Leu Lys Glu His Pro Asp Ala Trp Thr Arg Val Asp
       50                  55                  60
   Thr Ile Leu Glu Phe Ser Gln Asn Met Asn Thr Lys Tyr Tyr Gly Leu
   65                  70                  75                  80
   Gln Ile Leu Glu Asn Val Ile Lys Thr Arg Trp Lys Ile Leu Pro Arg
                   85                  90                  95
   Asn Gln Cys Glu Gly Ile Lys Lys Tyr Val Val Gly Leu Ile Ile Lys
               100                 105                 110
   Thr Ser Ser Asp Pro Thr Cys Val Glu Lys Glu Lys Val Tyr Ile Gly
           115                 120                 125
   Lys Leu Asn Met Ile Leu Val Gln Ile Leu Lys Gln Glu Trp Pro Lys
       130                 135                 140
   His Trp Pro Thr Phe Ile Ser Asp Ile Val Gly Ala Ser Arg Thr Ser
   145                 150                 155                 160
   Glu Ser Leu Cys Gln Asn Asn Met Val Ile Leu Lys Leu Leu Ser Glu
                   165                 170                 175
   Glu Val Phe Asp Phe Ser Ser Gly Gln Ile Thr Gln Val Lys Ser Lys
               180                 185                 190
   His Leu Lys Asp Ser Met Cys Asn Glu Phe Ser Gln Ile Phe Gln Leu
           195                 200                 205
   Cys Gln Phe Val Met Glu Asn Ser Gln Asn Ala Pro Leu Val His Ala
       210                 215                 220
   Thr Leu Glu Thr Leu Leu Arg Phe Leu Asn Trp Ile Pro Leu Gly Tyr
   225                 230                 235                 240
   Ile Phe Glu Thr Lys Leu Ile Ser Thr Leu Ile Tyr Lys Phe Leu Asn
                   245                 250                 255
   Val Pro Met Phe Arg Asn Val Ser Leu Lys Cys Leu Thr Glu Ile Ala
               260                 265                 270
   Gly Val Ser Val Ser Gln Tyr Glu Glu Gln Phe Val Thr Leu Phe Thr
           275                 280                 285
   Leu Thr Met Met Gln Leu Lys Gln Met Leu Pro Leu Asn Thr Asn Ile
       290                 295                 300
   Arg Leu Ala Tyr Ser Asn Gly Lys Asp Asp Glu Gln Asn Phe Ile Gln
   305                 310                 315                 320
   Asn Leu Ser Leu Phe Leu Cys Thr Phe Leu Lys Glu His Asp Gln Leu
                   325                 330                 335
   Ile Glu Lys Arg Leu Asn Leu Arg Glu Thr Leu Met Glu Ala Leu His
               340                 345                 350
   Tyr Met Leu Leu Val Ser Glu Val Glu Glu Thr Glu Ile Phe Lys Ile
           355                 360                 365
   Cys Leu Glu Tyr Trp Asn His Leu Ala Ala Glu Leu Tyr Arg Glu Ser
       370                 375                 380
   Pro Phe Ser Thr Ser Ala Ser Pro Leu Leu Ser Gly Ser Gln His Phe
   385                 390                 395                 400
   Asp Val Pro Pro Arg Arg Gln Leu Tyr Leu Pro Met Leu Phe Lys Val
                   405                 410                 415
   Arg Leu Leu Met Val Ser Arg Met Ala Lys Pro Glu Glu Val Leu Val
               420                 425                 430
   Val Glu Asn Asp Gln Gly Glu Val Val Arg Glu Phe Met Lys Asp Thr
           435                 440                 445
   Asp Ser Ile Asn Leu Tyr Lys Asn Met Arg Glu Thr Leu Val Tyr Leu
       450                 455                 460
   Thr His Leu Asp Tyr Val Asp Thr Glu Arg Ile Met Thr Glu Lys Leu
   465                 470                 475                 480
   His Asn Gln Val Asn Gly Thr Glu Trp Ser Trp Lys Asn Leu Asn Thr
                   485                 490                 495
   Leu Cys Trp Ala Ile Gly Ser Ile Ser Gly Ala Met His Glu Glu Asp
               500                 505                 510
   Glu Lys Arg Phe Leu Val Thr Val Ile Lys Asp Leu Leu Gly Leu Cys
           515                 520                 525
   Glu Gln Lys Arg Gly Lys Asp Asn Lys Ala Ile Ile Ala Ser Asn Ile
       530                 535                 540
   Met Tyr Ile Val Gly Gln Tyr Pro Arg Phe Leu Arg Ala His Trp Lys
   545                 550                 555                 560
   Phe Leu Lys Thr Val Val Asn Lys Leu Phe Glu Phe Met His Glu Thr
                   565                 570                 575
   His Asp Gly Val Gln Asp Met Ala Cys Asp Thr Phe Ile Lys Ile Ala
   580 585 590
   Gln Lys Cys Arg Arg His Phe Val Gln Val Gln Val Gly Glu Val Met
   595 600 605
   Pro Phe Ile Asp Glu Ile Leu Asn Asn Ile Asn Thr Ile Ile Cys Asp
   610 615 620
   Leu Gln Pro Gln Gln Val His Thr Phe Tyr Glu Ala Val Gly Tyr Met
   625 630 635 640
   Ile Gly Ala Gln Thr Asp Gln Thr Val Gln Glu His Leu Ile Glu Lys
   645 650 655
   Tyr Met Leu Leu Pro Asn Gln Val Trp Asp Ser Ile Ile Gln Gln Ala
   660 665 670
   Thr Lys Asn Val Asp Ile Leu Lys Asp Pro Glu Thr Val Lys Gln Leu
   675 680 685
   Gly Ser Ile Leu Lys Thr Asn Val Arg Ala Cys Lys Ala Val Gly His
   690 695 700
   Pro Phe Val Ile Gln Leu Gly Arg Ile Tyr Leu Asp Met Leu Asn Val
   705 710 715 720
   Tyr Lys Cys Leu Ser Glu Asn Ile Ser Ala Ala Ile Gln Ala Asn Gly
   725 730 735
   Glu Met Val Thr Lys Gln Pro Leu Ile Arg Ser Met Arg Thr Val Lys
   740 745 750
   Arg Glu Thr Leu Lys Leu Ile Ser Gly Trp Val Ser Arg Ser Asn Asp
   755 760 765
   Pro Gln Met Val Ala Glu Asn Phe Val Pro Pro Leu Leu Asp Ala Val
   770 775 780
   Leu Ile Asp Tyr Gln Arg Asn Val Pro Ala Ala Arg Glu Pro Glu Val
   785 790 795 800
   Leu Ser Thr Met Ala Ile Ile Val Asn Lys Leu Gly Gly His Ile Thr
   805 810 815
   Ala Glu Ile Pro Gln Ile Phe Asp Ala Val Phe Glu Cys Thr Leu Asn
   820 825 830
   Met Ile Asn Lys Asp Phe Glu Glu Tyr Pro Glu His Arg Thr Asn Phe
   835 840 845
   Phe Leu Leu Leu Gln Ala Val Asn Ser His Cys Phe Pro Ala Phe Leu
   850 855 860
   Ala Ile Pro Pro Thr Gln Phe Lys Leu Val Leu Asp Ser Ile Ile Trp
   865 870 875 880
   Ala Phe Lys His Thr Met Arg Asn Val Ala Asp Thr Gly Leu Gln Ile
   885 890 895
   Leu Phe Thr Leu Leu Gln Asn Val Ala Gln Glu Glu Ala Ala Ala Gln
   900 905 910
   Ser Phe Tyr Gln Thr Tyr Phe Cys Asp Ile Leu Gln His Ile Phe Ser
           915                 920                 925
   Val Val Thr Asp Thr Ser His Thr Ala Gly Leu Thr Met His Ala Ser
       930                 935                 940
   Ile Leu Ala Tyr Met Phe Asn Leu Val Glu Glu Gly Lys Ile Ser Thr
   945                 950                 955                 960
   Ser Leu Asn Pro Gly Asn Pro Val Asn Asn Gln Ile Phe Leu Gln Glu
                   965                 970                 975
   Tyr Val Ala Asn Leu Leu Lys Ser Ala Phe Pro His Leu Gln Asp Ala
               980                 985                 990
   Gln Val Lys Leu Phe Val Thr Gly Leu Phe Ser Leu Asn Gln Asp Ile
           995                 1000                1005
   Pro Ala Phe Lys Glu His Leu Arg Asp Phe Leu Val Gln Ile Lys Glu
       1010                1015                1020
   Phe Ala Gly Glu Asp Thr Ser Asp Leu Phe Leu Glu Glu Arg Glu Ile
   1025                1030                1035                1040
   Ala Leu Arg Gln Ala Asp Glu Glu Lys His Lys Arg Gln Met Ser Val
                   1045                1050                1055
   Pro Gly Ile Phe Asn Pro His Glu Ile Pro Glu Glu Met Cys Asp
               1060                1065                1070
<210> 18
   <211> 236
   <212> PRT
   <213> Mus musculus
<220>
   <223> Mouse CRM1 y2H
<400> 18
   Ala Arg Gly Gly Glu Val Met Pro Phe Ile Asp Glu Ile Leu Asn Asn
     1               5                  10                  15
   Ile Asn Thr Ile Ile Cys Asp Leu Gln Pro Gln Gln Val His Thr Phe
               20                  25                  30
   Tyr Glu Ala Val Gly Tyr Met Ile Gly Ala Gln Thr Asp Gln Thr Val
           35                  40                  45
   Gln Glu His Leu Ile Glu Lys Tyr Met Leu Leu Pro Asn Gln Val Trp
       50                  55                  60
   Asp Ser Ile Ile Gln Gln Ala Thr Lys Asn Val Asp Ile Leu Lys Asp
   65                  70                  75                  80
   Pro Glu Thr Val Lys Gln Leu Gly Ser Ile Leu Lys Thr Asn Val Arg
                   85                  90                  95
   Ala Cys Lys Ala Val Gly His Pro Phe Val Ile Gln Leu Gly Arg Ile
               100                 105                 110
   Tyr Leu Asp Met Leu Asn Val Tyr Lys Cys Leu Ser Glu Asn Ile Ser
           115                 120                 125
   Ala Ala Ile Gln Ala Asn Gly Glu Met Val Thr Lys Gln Pro Leu Ile
       130                 135                 140
   Arg Ser Met Arg Thr Val Lys Arg Glu Thr Leu Lys Leu Ile Ser Gly
   145                 150                 155                 160
   Trp Val Ser Arg Ser Asn Asp Pro Gln Met Val Ala Glu Asn Phe Val
                   165                 170                 175
   Pro Pro Leu Leu Asp Ala Val Leu Ile Asp Tyr Gln Arg Asn Val Pro
               180                 185                 190
   Ala Ala Arg Glu Pro Glu Val Leu Ser Thr Met Ala Ile Ile Val Asn
           195                 200                 205
   Lys Leu Gly Gly His Ile Thr Ala Glu Ile Pro Gln Ile Phe Asp Ala
       210                 215                 220
   Val Phe Glu Cys Thr Leu Asn Met Ile Asn Lys Asp
   225                 230                 235
<210> 19
   <211> 172
   <212> PRT
   <213> Mus musculus
<220>
   <223> Mouse TCTP NP_033455
<400> 19
   Met Ile Ile Tyr Arg Asp Leu Ile Ser His Asp Glu Leu Phe Ser Asp
   1 5 10 15
   Ile Tyr Lys Ile Arg Glu Ile Ala Asp Gly Leu Cys Leu Glu Val Glu
   20 25 30
   Gly Lys Met Val Ser Arg Thr Glu Gly Ala Ile Asp Asp Ser Leu Ile
   35 40 45
   Gly Gly Asn Ala Ser Ala Glu Gly Pro Glu Gly Glu Gly Thr Glu Ser
   50 55 60
   Thr Val Val Thr Gly Val Asp Ile Val Met Asn His His Leu Gln Glu
   65 70 75 80
   Thr Ser Phe Thr Lys Glu Ala Tyr Lys Lys Tyr Ile Lys Asp Tyr Met
   85 90 95
   Lys Ser Leu Lys Gly Lys Leu Glu Glu Gln Lys Pro Glu Arg Val Lys
   100 105 110
   Pro Phe Met Thr Gly Ala Ala Glu Gln Ile Lys His Ile Leu Ala Asn
   115 120 125
   Phe Asn Asn Tyr Gln Phe Phe Ile Gly Glu Asn Met Asn Pro Asp Gly
   130 135 140
   Met Val Ala Leu Leu Asp Tyr Arg Glu Asp Gly Val Thr Pro Phe Met
   145 150 155 160
   Ile Phe Phe Lys Asp Gly Leu Glu Met Glu Lys Cys
   165 170
<210> 20
   <211> 172
   <212> PRT
   <213> Homo sapiens
<220>
   <223> Human TCTP- NP_003286
<400> 20
   Met Ile Ile Tyr Arg Asp Leu Ile Ser His Asp Glu Leu Phe Ser Asp
   1 5 10 15
   Ile Tyr Lys Ile Arg Glu Ile Ala Asp Gly Leu Cys Leu Glu Val Glu
   20 25 30
   Gly Lys Met Val Ser Arg Thr Glu Gly Ala Ile Asp Asp Ser Leu Ile
   35 40 45
   Gly Gly Asn Ala Ser Ala Glu Gly Pro Glu Gly Glu Gly Thr Glu Ser
   50 55 60
   Thr Val Val Thr Gly Val Asp Ile Val Met Asn His His Leu Gln Glu
   65 70 75 80
   Thr Ser Phe Thr Lys Glu Ala Tyr Lys Lys Tyr Ile Lys Asp Tyr Met
   85 90 95
   Lys Ser Leu Lys Gly Lys Leu Glu Glu Gln Lys Pro Glu Arg Val Lys
   100 105 110
   Pro Phe Met Thr Gly Ala Ala Glu Gln Ile Lys His Ile Leu Ala Asn
   115 120 125
   Phe Asn Asn Tyr Gln Phe Phe Ile Gly Glu Asn Met Asn Pro Asp Gly
   130 135 140
   Met Val Ala Leu Leu Asp Tyr Arg Glu Asp Gly Val Thr Pro Phe Met
   145 150 155 160
   Ile Phe Phe Lys Asp Gly Leu Glu Met Glu Lys Cys
   165 170
<210> 21
   <211> 34
   <212> PRT
   <213> Mus musculus
<220>
   <223> Mouse TCTP y2H
<400> 21
   Asn Met Asn Pro Asp Gly Met Val Ala Leu Leu Asp Tyr Arg Glu Asp
   1 5 10 15
   Gly Val Thr Pro Phe Met Ile Phe Phe Lys Asp Gly Leu Glu Met Glu
   20 25 30
   Lys Cys
<210> 22
   <211> 204
   <212> PRT
   <213> Mus musculus
<220>
   <223> Tetraspan Net6 (SAS)- AF120265
<400> 22
   Met Val Cys Gly Gly Phe Ala Cys Ser Lys Asn Cys Leu Cys Ala Leu
   1               5                   10                  15
   Asn Leu Leu Tyr Thr Leu Val Ser Leu Leu Leu Ile Gly Ile Ala Ala
               20                  25                  30
   Trp Gly Ile Gly Phe Gly Leu Ile Ser Ser Leu Arg Val Val Gly Val
           35                  40                  45
   Val Ile Ala Val Gly Ile Phe Leu Phe Leu Ile Ala Leu Val Gly Leu
       50                  55                  60
   Ile Gly Ala Val Lys His His Gln Val Leu Leu Phe Phe Tyr Met Ile
   65                  70                  75                  80
   Ile Leu Leu Leu Val Phe Ile Val Gln Phe Ser Val Ser Cys Ala Cys
                   85                  90                  95
   Leu Ala Leu Asn Gln Glu Gln Gln Gly Gln Leu Leu Glu Val Gly Trp
               100                 105                 110
   Asn Asn Thr Ala Ser Ala Arg Asn Asp Ile Gln Arg Asn Leu Asn Cys
           115                 120                 125
   Cys Gly Phe Arg Ser Val Asn Pro Asn Asp Thr Cys Leu Ala Ser Cys
       130                 135                 140
   Val Lys Ser Asp His Ser Cys Ser Pro Cys Ala Pro Ile Ile Gly Glu
   145                 150                 155                 160
   Tyr Ala Gly Glu Val Leu Arg Phe Val Gly Gly Ile Gly Leu Phe Phe
                   165                 170                 175
   Ser Phe Thr Glu Ile Leu Gly Val Trp Leu Thr Tyr Arg Tyr Arg Asn
               180                 185                 190
   Gln Lys Asp Pro Arg Ala Asn Pro Ser Ala Phe Leu
           195                 200
<210> 23
   <211> 25
   <212> PRT
   <213> Mus musculus
<220>
   <223> Tetraspan Net6 (SAS)-y2H
<400> 23
   Glu Ile Leu Gly Val Trp Leu Thr Tyr Arg Tyr Arg Asn Gln Lys Asp
   1 5 10 15
   Pro Arg Ala Asn Pro Ser Ala Phe Leu
   20 25
<210> 24
   <211> 103
   <212> PRT
   <213> Mus musculus
<220>
   <223> Mouse ATPase H+ transporting mitochondrial protein-7304906
<400> 24
   Met Ala Lys Phe Ile Arg Asn Phe Ala Glu Lys Ala Pro Ser Met Val
   1 5 10 15
   Ala Ala Ala Val Thr Tyr Ser Lys Pro Arg Leu Ala Thr Phe Trp His
   20 25 30
   Tyr Ala Lys Val Glu Leu Val Pro Pro Thr Pro Ala Glu Ile Pro Thr
   35 40 45
   Ala Ile Gln Ser Val Lys Lys Met Ile Gln Ser Ala Lys Thr Gly Ser
   50 55 60
   Phe Lys His Leu Thr Val Lys Glu Ala Val Leu Asn Gly Leu Val Ala
   65 70 75 80
   Thr Glu Val Trp Met Trp Phe Tyr Ile Gly Glu Ile Ile Gly Lys Arg
   85 90 95
   Gly Ile Val Gly Tyr Asp Val
   100
<210> 25
   <211> 103
   <212> PRT
   <213> Homo sapiens
<220> <223> Human ATPase H+ transporting mitochondrial protein- AAD20961
<400> 25
   Met Ala Gln Phe Val Arg Asn Leu Val Glu Lys Thr Pro Ala Leu Val
   1 5 10 15
   Asn Ala Ala Val Thr Tyr Ser Lys Pro Arg Leu Ala Thr Phe Trp Tyr
   20 25 30
   Tyr Ala Lys Val Glu Leu Val Pro Pro Thr Pro Ala Glu Ile Pro Arg
   35 40 45
   Ala Ile Gln Ser Leu Lys Lys Ile Val Asn Ser Ala Gln Thr Gly Ser
   50 55 60
   Phe Lys Gln Leu Thr Val Lys Glu Ala Val Leu Asn Gly Leu Val Ala
   65 70 75 80
   Thr Glu Val Leu Met Trp Phe Tyr Val Gly Glu Ile Ile Gly Lys Arg
   85 90 95
   Gly Ile Ile Gly Tyr Asp Val
   100
<210> 26
   <211> 61
   <212> PRT
   <213> Mus musculus
<220>
   <223> Mouse ATPase H+ transporting mitochondrial protein-y2H
<400> 26
   Pro Ala Glu Ile Pro Thr Ala Ile Gln Ser Val Lys Lys Met Ile Gln
   1 5 10 15
   Ser Ala Lys Thr Gly Ser Phe Lys His Leu Thr Val Lys Glu Ala Val
   20 25 30
   Leu Asn Gly Leu Val Ala Thr Glu Val Trp Met Trp Phe Tyr Ile Gly
   35 40 45
   Glu Ile Ile Gly Lys Arg Gly Ile Val Gly Tyr Asp Val
   50 55 60
<210> 27
   <211> 69
   <212> PRT
   <213> Mus musculus
<220>
   <223> Mouse Cytochrome C Oxidase 8a (Cox8a)- 6680992
<400> 27
   Met Ser Val Leu Thr Pro Leu Leu Leu Arg Ser Leu Thr Gly Ser Ala
   1               5                   10                  15
   Arg Arg Leu Met Val Pro Arg Ala Gln Val His Ser Lys Pro Ala Arg
               20                  25                  30
   Glu Gln Leu Gly Val Leu Asp Ile Thr Ile Gly Leu Thr Ser Cys Phe
           35                  40                  45
   Val Cys Cys Leu Leu Pro Ala Gly Trp Val Leu Ser His Leu Glu Ser
       50                  55                  60
   Tyr Lys Lys Arg Glu
   65
<210> 28
   <211> 69
   <212> PRT
   <213> Homo sapiens
<220>
   <223> Human Mouse Cytochrome C Oxidase 8a (Cox8a)-XP_006132
<400> 28
   Met Ser Val Leu Thr Pro Leu Leu Leu Arg Gly Leu Thr Gly Ser Ala
   1               5                   10                  15
   Arg Arg Leu Pro Val Pro Arg Ala Lys Ile His Ser Leu Pro Pro Glu
               20                  25                  30
   Gly Lys Leu Gly Ile Met Glu Leu Ala Val Gly Leu Thr Ser Cys Phe
           35                  40                  45
   Val Thr Phe Leu Leu Pro Ala Gly Trp Ile Leu Ser His Leu Glu Thr
       50                  55                  60
   Tyr Arg Arg Pro Glu
   65
<210> 29
   <211> 53
   <212> PRT
   <213> Mus musculus
<220>
   <223> Mouse Cytochrome C Oxidase 8a (Cox8a)-y2H
<400> 29
   Arg Arg Leu Met Val Pro Arg Ala Gln Val His Ser Lys Pro Ala Arg
   1 5 10 15
   Glu Gln Leu Gly Val Leu Asp Ile Thr Ile Gly Leu Thr Ser Cys Phe
   20 25 30
   Val Cys Cys Leu Leu Pro Ala Gly Trp Val Leu Ser His Leu Glu Ser
   35 40 45
   Tyr Lys Lys Arg Glu
   50
<210> 30
   <211> 316
   <212> PRT
   <213> Mus musculus
<220>
   <223> Mouse Aldehyde-ketone reductase-6753037
<400> 30
   Met Ala Ser His Leu Glu Leu Asn Asn Gly Thr Lys Met Pro Thr Leu
   1               5                   10                  15
   Gly Leu Gly Thr Trp Lys Ser Pro Pro Gly Gln Val Thr Glu Ala Val
               20                  25                  30
   Lys Val Ala Ile Asp Leu Gly Tyr Arg His Ile Asp Cys Ser Gln Val
           35                  40                  45
   Tyr Gln Asn Glu Lys Glu Val Gly Val Ala Leu Gln Glu Lys Leu Lys
       50                  55                  60
   Glu Gln Val Val Lys Arg Gln Asp Leu Phe Ile Val Ser Lys Leu Trp
   65                  70                  75                  80
   Cys Thr Phe His Asp Lys Ser Met Val Lys Gly Ala Phe Gln Lys Thr
                   85                  90                  95
   Leu Ser Asp Leu Gln Leu Asp Tyr Leu Asp Leu Tyr Leu Ile His Trp
               100                 105                 110
   Pro Thr Gly Phe Lys Pro Gly Pro Asp Tyr Phe Pro Leu Asp Ala Ser
           115                 120                 125
   Gly Asn Val Ile Pro Ser Asp Thr Asp Phe Val Asp Thr Trp Thr Ala
       130                 135                 140
   Met Glu Gln Leu Val Asp Glu Gly Leu Val Lys Thr Ile Gly Val Ser
   145                 150                 155                 160
   Asn Phe Asn Pro Leu Gln Ile Glu Arg Ile Leu Asn Lys Pro Gly Leu
                   165                 170                 175
   Lys Tyr Lys Pro Ala Val Asn Gln Ile Glu Cys His Pro Tyr Leu Thr
               180                 185                 190
   Gln Glu Lys Leu Ile Glu Tyr Cys His Ser Lys Gly Ile Val Val Thr
           195                 200                 205
   Ala Tyr Ser Pro Leu Gly Ser Pro Asp Arg Pro Trp Ala Lys Pro Glu
       210                 215                 220
   Asp Pro Ser Leu Leu Glu Asp Pro Arg Ile Lys Ala Ile Ala Ala Lys
   225                 230                 235                 240
   Tyr Asn Lys Thr Thr Ala Gln Val Leu Ile Arg Phe Pro Ile Gln Arg
                   245                 250                 255
   Asn Leu Val Val Ile Pro Lys Ser Val Thr Pro Val Arg Ile Ala Glu
   260 265 270
   Asn Leu Lys Val Phe Asp Phe Glu Val Ser Ser Glu Asp Met Ala Thr
   275 280 285
   Leu Leu Ser Tyr Asn Arg Asn Trp Arg Val Cys Ala Leu Met Ser Cys
   290 295 300
   Ala Lys His Lys Asp Tyr Pro Phe His Ala Glu Val
   305 310 315
<210> 31
   <211> 176
   <212> PRT
   <213> Mus musculus
<220>
   <223> Mouse Aldehyde-ketone reductase-y2H
<400> 31
   Thr Trp Thr Ala Met Glu Gln Leu Val Asp Glu Gly Leu Val Lys Thr
   1 5 10 15
   Ile Gly Val Ser Asn Phe Asn Pro Leu Gln Ile Glu Arg Ile Leu Asn
   20 25 30
   Lys Pro Gly Leu Lys Tyr Lys Pro Ala Val Asn Gln Ile Glu Cys His
   35 40 45
   Pro Tyr Leu Thr Gln Glu Lys Leu Ile Glu Tyr Cys His Ser Lys Gly
   50 55 60
   Ile Val Val Thr Ala Tyr Ser Pro Leu Gly Ser Pro Asp Arg Pro Trp
   65 70 75 80
   Ala Lys Pro Glu Asp Pro Ser Leu Leu Glu Asp Pro Arg Ile Lys Ala
   85 90 95
   Ile Ala Ala Lys Tyr Asn Lys Thr Thr Ala Gln Val Leu Ile Arg Phe
   100 105 110
   Pro Ile Gln Arg Asn Leu Val Val Ile Pro Lys Ser Val Thr Pro Val
   115 120 125
   Arg Ile Ala Glu Asn Leu Lys Val Phe Asp Phe Glu Val Ser Ser Glu
   130 135 140
   Asp Met Ala Thr Leu Leu Ser Tyr Asn Arg Asn Trp Arg Val Cys Ala
   145 150 155 160
   Leu Met Ser Cys Ala Lys His Lys Asp Tyr Pro Phe His Ala Glu Val
   165 170 175
<210> 32
   <211> 215
   <212> PRT
   <213> Mus musculus
<220>
   <223> Mouse TRF-alpha (NAC)-U22151
<400> 32
   Met Pro Gly Glu Ala Thr Glu Thr Val Pro Ala Thr Glu Gln Glu Leu
   1               5                   10                  15
   Pro Gln Pro Gln Ala Glu Thr Gly Ser Gly Thr Glu Ser Asp Ser Asp
               20                  25                  30
   Glu Ser Val Pro Glu Leu Glu Glu Gln Asp Ser Thr Gln Thr Ala Thr
           35                  40                  45
   Gln Gln Ala Gln Leu Ala Ala Ala Ala Glu Ile Asp Glu Glu Pro Val
       50                  55                  60
   Ser Lys Ala Lys Gln Ser Arg Ser Glu Lys Lys Ala Arg Lys Ala Met
   65                  70                  75                  80
   Ser Lys Leu Gly Leu Arg Gln Val Thr Gly Val Thr Arg Val Thr Ile
                   85                  90                  95
   Arg Lys Ser Lys Asn Ile Leu Phe Val Ile Thr Lys Pro Asp Val Tyr
               100                 105                 110
   Lys Ser Pro Ala Ser Asp Thr Tyr Ile Val Phe Gly Glu Ala Lys Ile
           115                 120                 125
   Glu Asp Leu Ser Gln Gln Ala Gln Leu Ala Ala Ala Glu Lys Phe Lys
       130                 135                 140
   Val Gln Gly Glu Ala Val Ser Asn Ile Gln Glu Asn Thr Gln Thr Pro
   145                 150                 155                 160
   Thr Val Gln Glu Glu Ser Glu Glu Glu Glu Val Asp Glu Thr Gly Val
                   165                 170                 175
   Glu Val Lys Asp Ile Glu Leu Val Met Ser Gln Ala Asn Val Ser Arg
               180                 185                 190
   Ala Lys Ala Val Arg Ala Leu Lys Asn Asn Ser Asn Asp Ile Val Asn
           195                 200                 205
   Ala Ile Met Glu Leu Thr Met
       210                 215
<210> 33
   <211> 219
   <212> PRT
   <213> Mus musculus
<220>
   <223> Mouse TRF-alpha (NAC)-y2H
<400> 33
   Ser Pro His Lys Met Pro Gly Glu Ala Thr Glu Thr Val Pro Ala Thr
   1               5                   10                  15
   Glu Gln Glu Leu Pro Gln Pro Gln Ala Glu Thr Gly Ser Gly Thr Glu
               20                  25                  30
   Ser Asp Ser Asp Glu Ser Val Pro Glu Leu Glu Glu Gln Asp Ser Thr
           35                  40                  45
   Gln Thr Ala Thr Gln Gln Ala Gln Leu Ala Ala Ala Ala Glu Ile Asp
       50                  55                  60
   Glu Glu Pro Val Ser Lys Ala Lys Gln Ser Arg Ser Glu Lys Lys Ala
   65                  70                  75                  80
   Arg Lys Ala Met Ser Lys Leu Gly Leu Arg Gln Val Thr Gly Val Thr
                   85                  90                  95
   Arg Val Thr Ile Arg Lys Ser Lys Asn Ile Leu Phe Val Ile Thr Lys
               100                 105                 110
   Pro Asp Val Tyr Lys Ser Pro Ala Ser Asp Thr Tyr Ile Val Phe Gly
           115                 120                 125
   Glu Ala Lys Ile Glu Asp Leu Ser Gln Gln Ala Gln Leu Ala Ala Ala
       130                 135                 140
   Glu Lys Phe Lys Val Gln Gly Glu Ala Val Ser Asn Ile Gln Glu Asn
   145                 150                 155                 160
   Thr Gln Thr Pro Thr Val Gln Glu Glu Ser Glu Glu Glu Glu Val Asp
                   165                 170                 175
   Glu Thr Gly Val Glu Val Lys Asp Ile Glu Leu Val Met Ser Gln Ala
               180                 185                 190
   Asn Val Ser Arg Ala Lys Ala Val Arg Ala Leu Lys Asn Asn Ser Asn
           195                 200                 205
   Asp Ile Val Asn Ala Ile Met Glu Leu Thr Met
       210                 215
<210> 34
   <211> 204
   <212> PRT
   <213> Mus musculus
<220>
   <223> Mouse CGI-98-AF151856
<400> 34
   Met Ala Lys Val Gln Val Asn Asn Val Val Val Leu Asp Asn Pro Ser
    1              5                  10                  15
   Pro Phe Tyr Asn Pro Phe Gln Phe Glu Ile Thr Phe Glu Cys Ile Glu
               20                  25                  30
   Asp Leu Ser Glu Asp Leu Glu Trp Lys Ile Ile Tyr Val Gly Ser Ala
           35                  40                  45
   Glu Ser Glu Glu Tyr Asp Gln Val Leu Asp Ser Val Leu Val Gly Pro
       50                  55                  60
   Val Pro Ala Gly Arg His Met Phe Val Phe Gln Ala Asp Ala Pro Asn
   65                  70                  75                  80
   Pro Gly Leu Ile Pro Asp Ala Asp Ala Val Gly Val Thr Val Val Leu
                   85                  90                  95
   Ile Thr Cys Thr Tyr Arg Gly Gln Glu Phe Ile Arg Val Gly Tyr Tyr
               100                 105                 110
   Val Asn Asn Glu Tyr Thr Glu Thr Glu Leu Arg Glu Asn Pro Pro Val
           115                 120                 125
   Lys Pro Asp Phe Ser Lys Leu Gln Arg Asn Ile Leu Ala Ser Asn Pro
       130                 135                 140
   Arg Val Thr Arg Phe His Ile Asn Trp Glu Asp Asn Thr Glu Lys Leu
   145                 150                 155                 160
   Glu Asp Ala Glu Ser Ser Asn Pro Asn Leu Gln Ser Leu Leu Ser Thr
                   165                 170                 175
   Asp Ala Leu Pro Ser Ala Ser Lys Gly Trp Ser Thr Ser Glu Asn Ser
               180                 185                 190
   Leu Asn Val Met Leu Glu Ser His Met Asp Cys Met
           195                 200
<210> 35
   <211> 219
   <212> PRT
   <213> Mus musculus
<220>
   <223> Mouse CGI-98-y2H
<400> 35
   Ser Pro His Lys Met Pro Gly Glu Ala Thr Glu Thr Val Pro Ala Thr
   1 5 10 15
   Glu Gln Glu Leu Pro Gln Pro Gln Ala Glu Thr Gly Ser Gly Thr Glu
   20 25 30
   Ser Asp Ser Asp Glu Ser Val Pro Glu Leu Glu Glu Gln Asp Ser Thr
   35 40 45
   Gln Thr Ala Thr Gln Gln Ala Gln Leu Ala Ala Ala Ala Glu Ile Asp
   50 55 60
   Glu Glu Pro Val Ser Lys Ala Lys Gln Ser Arg Ser Glu Lys Lys Ala
   65 70 75 80
   Arg Lys Ala Met Ser Lys Leu Gly Leu Arg Gln Val Thr Gly Val Thr
   85 90 95
   Arg Val Thr Ile Arg Lys Ser Lys Asn Ile Leu Phe Val Ile Thr Lys
   100 105 110
   Pro Asp Val Tyr Lys Ser Pro Ala Ser Asp Thr Tyr Ile Val Phe Gly
   115 120 125
   Glu Ala Lys Ile Glu Asp Leu Ser Gln Gln Ala Gln Leu Ala Ala Ala
   130 135 140
   Glu Lys Phe Lys Val Gln Gly Glu Ala Val Ser Asn Ile Gln Glu Asn
   145 150 155 160
   Thr Gln Thr Pro Thr Val Gln Glu Glu Ser Glu Glu Glu Glu Val Asp
   165 170 175
   Glu Thr Gly Val Glu Val Lys Asp Ile Glu Leu Val Met Ser Gln Ala
   180 185 190
   Asn Val Ser Arg Ala Lys Ala Val Arg Ala Leu Lys Asn Asn Ser Asn
   195 200 205
   Asp Ile Val Asn Ala Ile Met Glu Leu Thr Met
   210 215
<210> 36
   <211> 412
   <212> ADN
   <213> Mus musculus
<220>
   <223> Mouse TSAP6int.EST.CL.11-BF579216
<400> 36
   ggcacgaggc ctcgtgccga attcgnnacg aggnnnngag agaactantn tcgangcatg 60
   catntagagg cccgcatcat gtaattatnt tatgtcactg cttacattca cagcattgng 120
   gnctttntgg ataantatcc tcccccaagt ttgggatgag actactanat gnngactggg 180
   ntntgacnct tgnttaagca ctttattaag ggcattgact tgngtggtgn ngtgggtaac 240
   ccccattctt ccacagcctg caagggaagg ncttccanaa aggctgtgga ctggggnttg 300
   ggngagacta gccannagtg agccctgcac ancaaggtaa tgggggttgn gcanagacag 360
   ncctagatct ggntttgtaa tgatttatnc nnnaaataaa nannccnnnn nc         412
<210> 37
   <211> 601
   <212> ADN
   <213> Mus musculus
<220>
   <223> Mouse TSAP6.EST/Cl.28(245)-BF470786
<400> 37
   ggcacgaggn anagaactaa cacttagang catgcatcta gagngccgna tcntgaannt 60
   attcctttgc tcacaaggtg ttgctttgtc ctcgtgatga gtttataagc cttcttccct 120
   ccctgctcct taacaaaaac aatgtgatgg atcggtgatg ttcacagttc tccctgtgcg 180
   gatgcacttg tgctcatagg gtttggtaag atgatctcaa gtaactacac cagctttggt 240
   gtagcagtga actccgggtg gtgctgggat ctgatccaac gaaaaacact tgccattctc 300
   accgaagaga actggctttg ggactctgtt aactttgggg aaggaaacat tgtatgtttg 360
   gctcagtgtg cttggctggt taatctttgg taatttcacc tgaatgagat ttgatgaatt 420
   aatgaatatt gaatttaaaa ctaattatga gtgaaaaata aaagggncgn gcttaaaaag 480
   gtatgtcacg ctnacnttca cgcacccccg tggggcacgt tccatgtgtt cncnggccng 540
   gacccaagcg tnacaggcat tacaactntg cccgagnctg agatggctct aaaaaaaaaa 600
   a                                                                 601
<210> 38
   <211> 256
   <212> ADN
   <213> Mus musculus
<220>
   <223> Mouse TSAP6intEST.cl.32(255)- AA611746
<400> 38
   ggcacgaggg tagcaattga aatgagaaat cagttcatca tttcaagtac tacattgcct 60
   gcattacaaa ttctctgatc acagaattgt acataaccct gagcctatgt acntgccatt 120
   tgattgtcag gttccatttg tgtcaaaacc taaagatagt gatgaccaag tgaaacagag 180
   tggtgtattg ctttttcata tgaatatttt cacataaaag catttgtgaa agcaaaaaaa 240
   aaaaaaaaaa aaaaaa                                                 256
<210> 39
   <211> 545
   <212> ADN
   <213> Mus musculus
<220>
   <223> Mouse TSAP6intEST.cl.40(279)- AI664118
<400> 39
   ggcacgaggc agtatctccc agtagcactt cttctggatt tgaattcaaa tgtcattctt 60
   aacatttctg ttgatgcttc tttgaaaatc ttcgcttgat tttggctgta aatcacctac 120
   cctgttaaat gcttgtttaa tcccaaggat gtaagttgag gtgaggtgtc ttctctgtct 180
   gccccattgc actcaactga atanaaatat ttggntttag agcatctcaa aagtgggtag 240
   aatgtccttt gggattttgt tgctccgttt aaaccaggtg tatngcgaca tgaacatgac 300
   tttcctaaag tggaaggatc tgtccccacc ttcacccagt ccttangaca agtagaaagg 360
   ggcancctgg cacttggcat aacctatgcc accanggcna gataactgtg aaaaagnggt 420
   atcctctgat gtcccncgta ttagnctcnn nttacaaanc acttaataaa actancctac 480
   tgtnnaacca ncnccaaaac cnacaaancc cccgcccctt ttttttnnan cggncccccn 540
   ntaga                                                             545
<210> 40
   <211> 477
   <212> ADN
   <213> Mus musculus
<220>
   <223> Mouse TSAP6int.EST.Cl.23(200)same as C1.40-AA915693
<400> 40
   ccagtagcac ttcttctgga tttgaattca aatgtcattc ttaacatttc tgttgatgct 60
   tctttgaaaa tcttcgcttg attttggctg taaatcacct accctgttaa atgctgttta 120
   atcccaagga tgtaagttga ggtgaggtgt cttctctgtc tgccccattg cactcaactg 180
   aatagaaata tttgggttta gagcatctca aaagtgggta gaatgtcctt tgggattttg 240
   ttgctccgtt taaaccaggt gtattgtgac atgaacatga ctttcctaaa gtggaaggat 300
   ctgtccccac cttcacccag tccttaggac aagtagaaag gggcattctg gcacttggca 360
   taacctatgc cacgatggtg agataatgtg aaaaagtggt atcctctgat gtcagcgtat 420
   tagtctcagt ttacaaagta cttaataaaa ctaactactg taaaaccaaa aaaaaaa    477
<210> 41
   <211> 733
   <212> ADN
   <213> Mus musculus
<220>
   <223> Mouse TSAP6int.EST.Cl.62(342)-AA537195
<400> 41
   gaattcggca cgagggtgtg cttggcctct gttagactcc tgtgtggttc cacaatgcct 60
   aagaaagccg gtgcgacaag caagggtaaa aaccaaacca aagaaccaga gacagcacct 120
   cctgctgcag ggcctgtagc aactgaccct aaaggttttg tcaccatagc cattcatgcc 180
   aaacctggct ccagacagaa cgctgtgaca gatctgagca ctgaggccgt tggtgtggcc 240
   attgcagcac ctccgtccca gggagaggca aatgcggagc tgtgtcggta cctctccaag 300
   gtcttggacc tcaggaagag tgacgtggtt ctggataagg gtggtaaatc tcgggaaaag 360
   gtggtgaagc tcttggcctc tacaactcca gaggaggtct tggagaagct gaaaacagaa 420
   gctgagaaga aataaaggcg agaaatggaa acagctgctg tctgcgacgg agcatctgag 480
   ctgcaggcac tgggagagga gctgaaggtc gccgcgtgga ggcggacagg acaccagctc 540
   cctggactcc gagtgaagct ttagctttat agctccacaa tgttttgtgg agttctgggg 600
   tctcctaatt cagtttacaa ctggaaaacc ttttgtgctg cttctattct aagtttttgg 660
   tttttaaatg caaaagtgtt ctctttatat cttttaaaga ttctctggac atttcgtgga 720
   aaagtaaata atc                                                    733
<210> 42
   <211> 611
   <212> ADN
   <213> Mus musculus
<220>
   <223> Mouse TSAP6int.EST.Cl.78(260)-AA137802
<400> 42
   gaattcggca cgaggnntan ctctaaaanc tgatntnngn ggcactatta naagntnnac 60
   tataaggagn ttctgnttgc cntgtaccta gcacttcccc agatgngcan ataacggagt 120
   agncaggtgg aatgcactaa tgcacccagc attcatgtgg ggtangagga gcgggnaggt 180
   caaagtggnc cttacaaata gcaggctcta ngccagggtg gnctatagat gactctgnnt 240
   ctaacaaaca cacaaataaa acattgtgtg aatgatggta ttttagtaca tggttctcta 300
   cccagcccct ctgtccctca ngcattctan actctgtgct gacaggacta aattgctcct 360
   acaaaaataa tagtactaan gtataancca attctatatt tgtgatagaa naaaaaatga 420
   atattcttta acaggaagta tgttcctaga atgggatatc atatggtaaa ttacagtgat 480
   aacaatagat ctggttgaca tgacgttata tttttgtgaa gtatttcana agaatatata 540
   ttttctcagt tttataaaaa tgaaaaataa aaganattgg ttgtttataa aaaaaaaaaa 600
   aaanaaaaaa a                                                      611
<210> 43
   <211> 548
   <212> ADN
   <213> Mus musculus
<220>
   <223> Mouse TSAP6int.EST.Cl.81-BE627851
<400> 43
   gaattcggca cgagggagag agagaactan tctcgaggca tgcatctaga ggnccgcatc 60
   atgtaattag ntatgtcacg cttcnttcac gcagtcatat ttcataagct cttttggcct 120
   gggtacagca gaaaactagc agttttccta cttggcgcaa gtgacttgtg cctccttgtc 180
   tgtcctttgt cagcacaggt gcaggcaaac ccttccagct gggttaattg ccgtttatgg 240
   tttactcact tagggaaagg gcttaggtgt taggctgtgg gctgcttctc cctaaccatc 300
   cattgtgcag acttctcatc taaaaaggtt ggtggctttt gcttgggatc agtgctctgc 360
   taacgccctt gctggtctct ccacacattc ctgtcattga gacttgaatt gtaggtgtga 420
   tgttatgcac aggatgctca gagctatgtt actactattc ttagtttgta aattgtcctt 480
   ttgataccat cttgttttct tttgtaggta taaataaata cttgacaata aaaaaaaaaa 540
   aaaaaaaa                                                          548
<210> 44
   <211> 338
   <212> PRT
   <213> Mus musculus
<220>
   <223> Mouse TSAP6int. HUKIAA0095-11435026
<400> 44
<210> 45
   <211> 75
   <212> PRT
   <213> Mus musculus
<220>
   <223> Mouse TSAP6int. HUKIAA0095-y2H
<400> 45
   Leu Ser Glu Val Leu Leu Ala Thr Met Asn Ile Leu Phe Thr Gln Phe
   1               5                   10                  15
   Lys Arg Leu Lys Gly Thr Ser Pro Ser Ser Ser Ser Arg Pro Gln Arg
               20                  25                  30
   Val Ile Glu Asp Arg Asp Ser Gln Leu Arg Ser Gln Ala Arg Thr Leu
           35                  40                  45
   Ile Thr Phe Ala Gly Met Ile Pro Tyr Arg Thr Ser Gly Asp Thr Asn
       50                  55                  60
   Ala Arg Leu Val Gln Met Glu Val Leu Met Asn
   65                  70                  75
<210> 46
   <211> 147
   <212> ADN
   <213> Mus musculus
<220>
   <223> Mouse Sca-1: Non-coding region: NM_008529
<400> 46
   gttcaggagg ctgcccatgg aggactgcca cccctccaga tgaaggctcc cactacccga 60
   tgcagttgag tcccatcctg ccctctctgc ccacactggc ttcctgctgc tattctagtg 120
   cctcaaataa accgttcaca cccttaa 147
<210> 47
   <211> 50
   <212> PRT
   <213> Mus musculus
<220>
   <223> Xaa = codon stop
<220>
   <223> Mouse Sca-1 peptide
<400> 47
   Val Gln Glu Ala Ala His Gly Gly Leu Pro Pro Leu Gln Met Lys Ala
   1 5 10 15
   Pro Thr Thr Arg Cys Ser Xaa Val Pro Ser Cys Pro Leu Cys Pro His
   20 25 30
   Trp Leu Pro Ala Ala Ile Leu Val Pro Gln Ile Asn Arg Ser His Pro
   35 40 45
   Xaa Lys
   50
<210> 48
   <211> 133
   <212> ADN
   <213> Mus musculus
<220>
   <223> Mouse Nebulin related anchoring protein: U76618
<400> 48
   ggcacgaggc ttttggatat atttttaatt aaaaaaatca catgagaaat gtaatctgta 60
   tagtgatact ttaataagca catactcaat aaacaactgc aatgatgtcg acagcaaaaa 120
   aaaaaaaaaa aaa 133
<210> 49
   <211> 2969
   <212> ADN
   <213> Mus musculus
<220>
   <223> Murine sequence TSAP6
<400> 49
   cctcggaaac ttaggtgaat ccacttctag tccagacctt tgaggtggga agacacacct 60
   ttaatctggg ccacaccttc tgctgggagg tataaggacg cagacctgct ctttgcctgc 120
   ttgtcctcac cttgctaaca catccactcc tcactggcac tggggctact tctttgggat 180
   tccagcatat actgaagacc agctgaaata tctagcctca tagactgaac aactactgga 240
   tttttggact ttctgttcac agtcaggtat actgcagcct tgagctccag ccacccagct 300
   tcagaagaaa tggctgcaga ggcccacagg cagcagggct cttgccccac catcccctca 360
   gagggctgtg gaaagtcacc agagaagaaa ggcagtgcgg ccgactctag acctggcact 420
   gctatgtcgg gggagatgga caagccgctg atcagccgcc gcctagtgga cagtgatggc 480
   agtctggctg aggtccccaa ggaggccccc aaagtgggca tcctgggcag tggggatttt 540
   gcccgttccc tggccacacg cctggtgggc tctggcttca gtgtggtggt ggggagccgt 600
   aaccccaaac gcacggctgg cctcttcccc tccttagctc aagtgacttt ccaggaggaa 660
   gccgtgagct ctccagaggt catctttgtg gccgtgttcc gggagcacta ctcctcactg 720
   tgcagtcttg ctgaccagtt ggctggcaag atcctcgtgg atgtaagcaa ccccacggag 780
   aaggagcatc ttcagcaccg ccagtctaat gctgagtacc tggcctcact ctttcctgcg 840
   tgcactgtgg tgaaggcctt caacgtcatc tctgcatggg ccctacaggc tggcccaagg 900
   gatgggaaca ggcaggtgct catctgcagt gatcagccag aagccaagcg caccatctca 960
   gagatggcac gcgccatggg tttcacaccc ctggacatgg gatccctggc ctcagcgagg 1020
   gaggtagaag ccatacccct gcgcctcctt ccatcctgga aggtgcccac cctcctggca 1080
   ctggggctct ttgtgtgctt ctacacctac aacttcatcc gggacgttct acagccatac 1140
   attcggaaag atgagaacaa gttctacaag atgcccttgt ctgtggtcaa caccacacta 1200
   ccctgtgtgg cttatgtgct gctgtcccta gtgtacctgc ccggtgtgct ggcagctgcg 1260
   cttcagctgc ggagggggac caagtaccag cgcttcccag actggctgga ccactggctg 1320
   cagcatcgca agcagatcgg gctgctcagc ttcttcttcg cgatgctgca cgctctctac 1380
   agcttctgcc tgccgctgcg ccgctcccac cgctacgacc tggtcaatct ggctgtgaag 1440
   caggtcctgg ccaacaagag ccgcctctgg gttgaggaag aagtctggag gatggagata 1500
   tacctgtccc tgggtgtgct ggccctgggc atgttgtcgc tgctggctgt cacctcgctc 1560
   ccgtccattg ccaactccct caactggaag gagttcagct tcgtgcagtc cacgctgggc 1620
   ttcgtggccc tgatactcag cacaatgcac acactcacct acggctggac ccgtgccttt 1680
   gaggaaaacc actacaagtt ctacctgccg cccacattca cactcacgct gctcctgccc 1740
   tgtgtgatca tcctggccaa gggcctcttc ctcctgccct gcctcagccg cagactcacc 1800
   aagatccgca ggggctggga gaaagatggg gctgtcaagt tcatgctgcc cggcgaccac 1860
   acacaggggg agaaaacaag ccacgtgtga ggccctggaa gcggagatgg cttgtggggg 1920
   ccctgagctg ggttcgggtc tcttttctgg atgctgcaca gcgaggtgat gatatatgcg 1980
   tgggtggctg agatcctaat tcctggggtg caggtgtaaa ctgacatact cagaatgaca 2040
   ccccatacat gtgatatgta ctcacatata tttcacatat aataagattt gctattattc 2100
   ttacttagct aaaaaaaaaa agtgggtccc tatatttcag cgtaagcatt tcaaagcaaa 2160
   tgccacacat tgaacagcag atcccaccct tgtggtatct acagaggcag acagacactc 2220
   tggtatagga gaaactgtct ttcgttggat tctctccttt aatctctatg ctccttatta 2280
   gctgaatcct aaagttggtg caaagctggg gcaagaaatg cctctggtgc cacctacccc 2340
   catcccaggg ctaagaaaga agcctcgagt gaacagggaa ccaggtctgg actctgctgc 2400
   ttccctgggc gtgcgtgggg aggctcagca agaccccctg ggatctatgc aggagctttt 2460
   tcaggtccgt cctttcttca gggaagggtc tgaagctgcc ccatctgatc ctagctgagc 2520
   tgagaagatt cttccccacc ccctgaaagt ccagagtcac caccggagcc tgcaaattga 2580
   tccttctgcg aaggtgtgaa gtcaccgcct ctccagagcc attaatgaac ctggtcttcg 2640
   ggaggaggat aattgtttcc tctccattaa gttgctggtg accccccttt aaatcactgt 2700
   gccttctcgc cttttccatc attaatttgg acatctccgt ggagtggaca cttgtctggg 2760
   cagttcgggg ggggggggag cattagagat tgcagagaat aaccatcgaa tcctcttctt 2820
   ggggcaaccc tccccttgga tgtgccccag gcctgccttc attaaattgg tccctgagga 2880
   gaaaaaaaaa aaaaaaaaaa aaaaaaaaaa aaaaaaaaaa aaaaaattta aaaaaaaaaa 2940
   aaaaaaaaaa aaaaaaaaaa aaaaaaaaa 2969
<210> 50
   <211> 526
   <212> PRT
   <213> Mus musculus
<220>
   <223> Murine TSAP6 protein
<400> 50
   Met Ala Ala Glu Ala His Arg Gln Gln Gly Ser Cys Pro Thr Ile Pro
   1 5 10 15
   Ser Glu Gly Cys Gly Lys Ser Pro Glu Lys Lys Gly Ser Ala Ala Asp
   20 25 30
   Ser Arg Pro Gly Thr Ala Met Ser Gly Glu Met Asp Lys Pro Leu Ile
   35 40 45
   Ser Arg Arg Leu Val Asp Ser Asp Gly Ser Leu Ala Glu Val Pro Lys
   50 55   60
   Glu Ala Pro Lys Val Gly Ile Leu Gly Ser Gly Asp Phe Ala Arg Ser
   65 70 75 80
   Leu Ala Thr Arg Leu Val Gly Ser Gly Phe Ser Val Val Val Gly Ser
   85 90 95
   Arg Asn Pro Lys Arg Thr Ala Gly Leu Phe Pro Ser Leu Ala Gln Val
   100 105 110
   Thr Phe Gln Glu Glu Ala Val Ser Ser Pro Glu Val Ile Phe Val Ala
   115 120 125
   Val Phe Arg Glu His Tyr Ser Ser Leu Cys Ser Leu Ala Asp Gln Leu
   130 135 140
   Ala Gly Lys Ile Leu Val Asp Val Ser Asn Pro Thr Glu Lys Glu His
   145 150 155 160
   Leu Gln His Arg Gln Ser Asn Ala Glu Tyr Leu Ala Ser Leu Phe Pro
   165 170 175
   Ala Cys Thr Val Val Lys Ala Phe Asn Val Ile Ser Ala Trp Ala Leu
   180 185 190
   Gln Ala Gly Pro Arg Asp Gly Asn Arg Gln Val Leu Ile Cys Ser Asp
   195 200 205
   Gln Pro Glu Ala Lys Arg Thr Ile Ser Glu Met Ala Arg Ala Met Gly
   210 215 220
   Phe Thr Pro Leu Asp Met Gly Ser Leu Ala Ser Ala Arg Glu Val Glu
   225 230 235 240
   Ala Ile Pro Leu Arg Leu Leu Pro Ser Trp Lys Val Pro Thr Leu Leu
   245 250 255
   Ala Leu Gly Leu Phe Val Cys Phe Tyr Thr Tyr Asn Phe Ile Arg Asp
   260 265 270
   Val Leu Gln Pro Tyr Ile Arg Lys Asp Glu Asn Lys Phe Tyr Lys Met
   275 280 285
   Pro Leu Ser Val Val Asn Thr Thr Leu Pro Cys Val Ala Tyr Val Leu
   290 295 300
   Leu Ser Leu Val Tyr Leu Pro Gly Val Leu Ala Ala Ala Leu Gln Leu
   305 310 315 320
   Arg Arg Gly Thr Lys Tyr Gln Arg Phe Pro Asp Trp Leu Asp His Trp
   325 330 335
   Leu Gln His Arg Lys Gln Ile Gly Leu Leu Ser Phe Phe Phe Ala Met
   340 345 350
   Leu His Ala Leu Tyr Ser Phe Cys Leu Pro Leu Arg Arg Ser His Arg
   355 360 365
   Tyr Asp Leu Val Asn Leu Ala Val Lys Gln Val Leu Ala Asn Lys Ser
   370                 375                 380
   Arg Leu Trp Val Glu Glu Glu Val Trp Arg Met Glu Ile Tyr Leu Ser
   385                 390                 395                 400
   Leu Gly Val Leu Ala Leu Gly Met Leu Ser Leu Leu Ala Val Thr Ser
                   405                 410                 415
   Leu Pro Ser Ile Ala Asn Ser Leu Asn Trp Lys Glu Phe Ser Phe Val
               420                 425                 430
   Gln Ser Thr Leu Gly Phe Val Ala Leu Ile Leu Ser Thr Met His Thr
           435                 440                 445
   Leu Thr Tyr Gly Trp Thr Arg Ala Phe Glu Glu Asn His Tyr Lys Phe
       450                 455                 460
   Tyr Leu Pro Pro Thr Phe Thr Leu Thr Leu Leu Leu Pro Cys Val Ile
   465                 470                 475                 480
   Ile Leu Ala Lys Gly Leu Phe Leu Leu Pro Cys Leu Ser Arg Arg Leu
                   485                 490                 495
   Thr Lys Ile Arg Arg Gly Trp Glu Lys Asp Gly Ala Val Lys Phe Met
               500                 505                 510
   Leu Pro Gly Asp His Thr Gln Gly Glu Lys Thr Ser His Val
           515                 520                 525
<210> 51
   <211> 1720
   <212> ADN
   <213> Homo sapiens
<220>
   <223> Human sequence TSAP6
<400> 51
   acccagccac caaaatgcca gaagagatgg acaagccact gatcagcctc cacctggtgg 60
   acagcgatag tagccttgcc aaggtccccg atgaggcccc caaagtgggc atcctgggta 120
   gcggggactt tgcccgctcc ctggccacac gcctggtggg ctctggcttc aaagtggtgg 180
   tggggagccg caaccccaaa cgcacagcca ggctgtttcc ctcagcggcc caagtgactt 240
   tccaagagga ggcagtgagc tccccggagg tcatctttgt ggctgtgttc cgggagcact 300
   actcttcact gtgcagtctc agtgaccagc tggcgggcaa gatcctggtg gatgtgagca 360
   accctacaga gcaagagcac cttcagcatc gtgagtccaa tgctgagtac ctggcctccc 420
   tcttccccac ttgcacagtg gtcaaggcct tcaatgtcat ctctgcctgg accctgcagg 480
   ctggcccaag ggatggtaac aggcaggtgc ccatctgcgg tgaccagcca gaagccaagc 540
   gtgctgtctc ggagatggcg ctcgccatgg gcttcatgcc cgtggacatg ggatccctgg 600
   cgtcagcctg ggaggtggag gccatgcccc tgcgcctcct cccggcctgg aaggtgccca 660
   ccctgctggc cctggggctc ttcgtctgct tctatgccta caacttcgtc cgggacgttc 720
   tgcagcccta tgtgcaggaa agccagaaca agttcttcaa gctgcccgtg tccgtggtca 780
   acaccacact gccgtgcgtg gcctacgtgc tgctgtcact cgtgtacttg cccggcgtgc 840
   tggcggctgc cctgcagctg cggcgcggca ccaagtacca gcgcttcccc gactggctgg 900
   accactggct acagcaccgc aagcagatcg ggctgctcag cttcttctgc gccgccctgc 960
   acgccctcta cagcttctgc ttgccgctgc gccgcgccca ccgctacgac ctggtcaacc 1020
   tggcagtcaa gcaggtcttg gccaacaaga gccacctctg ggtggaggag gaggtctggc 1080
   ggatggagat ctacctctcc ctgggagtgc tggccctcgg cacgttgtcc ctgctggccg 1140
   tgacctcact gccgtccatt gcaaactcgc tcaactggag ggagttcagc ttcgttcagt 1200
   cctcactggg ctttgtggcc ctcgtgctga gcacactgca cacgctcacc tacggctgga 1260
   cccgcgcctt cgaggagagc cgctacaagt tctacctgcc tcccaccttc acgctcacgc 1320
   tgctggtgcc ctgcgtcgtc atcctggcca aagccctgtt tctcctgccc tgcatcagcc 1380
   gcagactcgc caggatccgg agaggctggg agagggagag caccatcaag ttcacgctgc 1440
   ccacagacca cgccctggcc gagaagacga gccacgtatg aggtgcctgc cctgggctct 1500
   ggaccccggg cacacgaggg acggtgccct gagcccgtta ggtttctttt tcttggtggt 1560
   gcaaagtggt ataactgtgt gcaaatagga ggtttgaggt ccaaattcct gggactcaaa 1620
   tgtatgcagt actattcaga atgatataca cacatatgtg tatatgtatt tacatatatt 1680
   ccacatatat aacaggattt gcaattatac atagctagct                       1720
<210> 52
   <211> 488
   <212> PRT
   <213> Homo sapiens
<220>
   <223> Human TSAP6 protein
<400> 52
   Met Pro Glu Glu Met Asp Lys Pro Leu Ile Ser Leu His Leu Val Asp
   1 5 10 15
   Ser Asp Ser Ser Leu Ala Lys Val Pro Asp Glu Ala Pro Lys Val Gly
   20 25 30
   Ile Leu Gly Ser Gly Asp Phe Ala Arg Ser Leu Ala Thr Arg Leu Val
   35 40 45
   Gly Ser Gly Phe Lys Val Val Val Gly Ser Arg Asn Pro Lys Arg Thr
   50 55 60
   Ala Arg Leu Phe Pro Ser Ala Ala Gln Val Thr Phe Gln Glu Glu Ala
   65 70 75 80
   Val Ser Ser Pro Glu Val Ile Phe Val Ala Val Phe Arg Glu His Tyr
   85 90 95
   Ser Ser Leu Cys Ser Leu Ser Asp Gln Leu Ala Gly Lys Ile Leu Val
   100 105 110
   Asp Val Ser Asn Pro Thr Glu Gln Glu His Leu Gln His Arg Glu Ser
   115 120 125
   Asn Ala Glu Tyr Leu Ala Ser Leu Phe Pro Thr Cys Thr Val Val Lys
   130 135 140
   Ala Phe Asn Val Ile Ser Ala Trp Thr Leu Gln Ala Gly Pro Arg Asp
   145 150 155 160
   Gly Asn Arg Gln Val Pro Ile Cys Gly Asp Gln Pro Glu Ala Lys Arg
   165 170 175
   Ala Val Ser Glu Met Ala Leu Ala Met Gly Phe Met Pro Val Asp Met
   180 185 190
   Gly Ser Leu Ala Ser Ala Trp Glu Val Glu Ala Met Pro Leu Arg Leu
   195 200 205
   Leu Pro Ala Trp Lys Val Pro Thr Leu Leu Ala Leu Gly Leu Phe Val
   210 215 220
   Cys Phe Tyr Ala Tyr Asn Phe Val Arg Asp Val Leu Gln Pro Tyr Val
   225 230 235 240
   Gln Glu Ser Gln Asn Lys Phe Phe Lys Leu Pro Val Ser Val Val Asn
   245 250 255
   Thr Thr Leu Pro Cys Val Ala Tyr Val Leu Leu Ser Leu Val Tyr Leu
   260 265 270
   Pro Gly Val Leu Ala Ala Ala Leu Gln Leu Arg Arg Gly Thr Lys Tyr
   275 280 285
   Gln Arg Phe Pro Asp Trp Leu Asp His Trp Leu Gln His Arg Lys Gln
   290 295 300
   Ile Gly Leu Leu Ser Phe Phe Cys Ala Ala Leu His Ala Leu Tyr Ser
   305 310 315 320
   Phe Cys Leu Pro Leu Arg Arg Ala His Arg Tyr Asp Leu Val Asn Leu
   325 330 335
   Ala Val Lys Gln Val Leu Ala Asn Lys Ser His Leu Trp Val Glu Glu
   340 345 350
   Glu Val Trp Arg Met Glu Ile Tyr Leu Ser Leu Gly Val Leu Ala Leu
   355 360 365
   Gly Thr Leu Ser Leu Leu Ala Val Thr Ser Leu Pro Ser Ile Ala Asn
   370 375 380
   Ser Leu Asn Trp Arg Glu Phe Ser Phe Val Gln Ser Ser Leu Gly Phe
   385 390 395 400
   Val Ala Leu Val Leu Ser Thr Leu His Thr Leu Thr Tyr Gly Trp Thr
   405 410 415
   Arg Ala Phe Glu Glu Ser Arg Tyr Lys Phe Tyr Leu Pro Pro Thr Phe
   420 425 430
   Thr Leu Thr Leu Leu Val Pro Cys Val Val Ile Leu Ala Lys Ala Leu
   435 440 445
   Phe Leu Leu Pro Cys Ile Ser Arg Arg Leu Ala Arg Ile Arg Arg Gly
   450 455 460
   Trp Glu Arg Glu Ser Thr Ile Lys Phe Thr Leu Pro Thr Asp His Ala
   465 470 475 480
   Leu Ala Glu Lys Thr Ser His Val
   485

## Description

La présente invention a pour objet des méthodes de détection, identification et/ou criblage de composés pouvant notamment être utilisés pour le traitement des cancers ou de certaines maladies neuro-dégénéràtives, liés à des dérèglements dans la régulation de la réversion/suppression tumorale et/ou de l'apoptose, dans la chaîne biologique de p53.

L'apoptose, ou mort cellulaire, est un phénomène complexe qui est régulé par de nombreuses protéines, dont la protéine p53. cette protéine interagit avec de nombreuses autres protéines, et son expression, qui induit les phénomènes de mort cellulaire et de réversion tumorale, peut être corrélée avec l'induction ou la répression de l'expression d'autres gènes cellulaires.

Les inventeurs de la présente invention ont ainsi mis en évidence des gènes induits et activés lors de la cascade menant à la réversion tumorale et/ou l'apoptose (TSAP pour « Tumor Suppressor Activated Pathway»), ou des gènes réprimés (TSIP pour «Tumor Suppressor Inhibited Pathway»). Ces gènes ont fait notamment l'objet des demandes de brevets WO 97/22695 ou WO 00/08147.

Il est important de pouvoir comprendre précisément les mécanismes de la cascade de p53, afin de pouvoir générer de nouveaux composés ayant une activité antitumorale (pouvant notamment induire l'apoptose ou la suppression tumorale), ou pouvant être utilisés pour le traitement de maladies neuro-dégénératives. En effet, les inventeurs de la présente demande ont démontré que la préséniline 1 (PS1), dont la rôle avait été suggéré dans la maladie d'Alzheimer, était identique à la protéine TSIP 2 décrite dans la demande WO 97/22695. Ainsi, il est légitime de rechercher des médicaments pouvant interférer dans l'apoptose, afin de réduire ce phénomène, et qui pourraient être utilisés dans les maladies neuro-dégénératives.

La présente invention se rapporte à des procédés de criblage et d'identification de produits pouvant interférer dans la cascade de p53, et ainsi induire la réversion tumorale et/ou l'apoptose ou inversement, diminuer les phénomènes d'apoptose. Amson, R.B. et al. suggère un rôle pour TSAP6 sur l'effet apoptotique de P53 sans préciser lequel.

La présente invention est basée sur les interactions de la protéine TSAP 6 avec d'autres protéines, telles que mises en évidence par les inventeurs de la présente demande. La protéine TSAP 6, décrite dans la demande de brevet WO 97/22695, et dans GenBank sous le numéro U50961, est une protéine présentant six domaines transmembranaires, ce qui suggère une localisation dans une membrane cellulaire. Ainsi, il est raisonnable de penser que la protéine TSAP6 peut agir comme un récepteur dans le métabolisme de régulation de l'apoptose liée à p53, et que la détermination de ses partenaires de liaison peut s'avérer importante pour ce qui est de la compréhension globale de la régulation de l'apoptose/réversion tumorale dans les cellules.

La séquence nucléotidique de TSAP6 murine est représentée par SEQ ID N° 49 et le cadre ouvert de lecture de la protéine correspond à SEQ ID N° 50.

La séquence nucléotidique de TSAP6 humaine est représentée par SEQ ID N° 51 et le cadre ouvert de lecture de la protéine correspond à SEQ ID N° 52.

Ainsi, dans un premier mode de mise en oeuvre, la présente invention se rapporte à des procédés de criblage et/ou de sélection ou d'identification d'un composé interférant avec, réduisant ou inhibant la liaison de TSAP6 à l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, présentant les étapes de :
a) mise en contact dudit composé avec un système permettant la détermination *in vitro* de la liaison entre TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 ;
b) identification de la diminution et/ou l'inhibition de la liaison entre TSAP6 et ladite protéine choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48.

Afin de déterminer des composés permettant l'augmentation de la réversion tumorale et/ou la mort cellulaire (apoptose), il est également possible de définir un procédé selon l'invention, notamment un procédé de criblage, de sélection ou d'identification de composés ayant pour fonction une augmentation de la réversion tumorale et/ou la mort cellulaire (apoptose), présentant les étapes de :
a) mise en contact dudit composé avec un système permettant la détermination *in vitro* de la liaison entre TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 ;
b) identification des composés induisant la diminution et/ou l'inhibition de la liaison entre TSAP6 et ladite protéine définie en a) ;
c) mise en contact des composés sélectionnés dans l'étape b) dans un système in vitro permettant de mesurer les phénomènes d'apoptose et/ou de réversion tumorale ;
d) identification de l'augmentation de la réversion tumorale et/ou de la mort cellulaire (apoptose) dans ledit modèle par comparaison avec un modèle témoin avec laquelle ledit composé n'a pas été mis en contact.

Un tel procédé est donc également un objet de la présente invention.

La présente invention a également pour objet un procédé de criblage, de sélection ou d'identification de composés ayant pour fonction une diminution et/ou l'inhibition de la réversion tumorale et/ou la mort cellulaire (apoptose), présentant les étapes de :
a) mise en contact dudit composé avec un système permettant la détermination *in vitro* de la liaison entre TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 ;
b) identification des composés induisant la diminution et/ou l'inhibition de la liaison entre TSAP6 et ladite protéine définie en a) ;
c) mise en contact des composés sélectionnés dans l'étape b) dans un système in vitro permettant de mesurer les phénomènes d'apoptose et/ou de réversion tumorale ;
d) identification de la diminution et/ou l'inhibition de la réversion tumorale et/ou de la mort cellulaire (apoptose) dans ledit modèle par comparaison avec un modèle témoin avec laquelle ledit composé n'a pas été mis en contact.

La présente invention utilise donc le fait que les protéines TSAP6 et les protéines choisies parmi SEQ ID N° 1 à SEQ ID N° 35 ou SEQ ID N° 44 ou 46, ou codées par un acide nucléique choisi parmi SEQ ID N° 36 à SEQ ID N° 43 ou SEQ ID N° 45 ou 47 peuvent se lier l'une à l'autre. Il est donc intéressant d'identifier les domaines de chaque protéine qui sont effectivement en contact avec l'autre protéine. En effet, ceci devrait permettre de pouvoir utiliser les peptides ainsi identifiés en tant que leurres ou agonistes des protéines complètes. Ceci peut ainsi permettre de définir des composés qui interféreront dans la liaison TSAP6 - une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, et qui pourront soit induire la suppression tumorale et/ou l'apoptose, soit à l'inverse diminuer ces phénomènes.

La présente invention a donc notamment pour objet un procédé d'identification d'une région de TSAP6 se liant avec l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, comportant les étapes de :
a) mise en contact de peptides issus de la protéine TSAP6 dans un système permettant d'évaluer in vitro la liaison de la protéine TSAP6 avec ladite protéine choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 ;
b) identification des peptides induisant la diminution de la liaison entre la protéine TSAP 6 et ladite protéine définie en a) par comparaison avec la liaison observée entre TSAP6 et ladite protéine définie en a), lorsque lesdits peptides sont absents du système.

Par « région de TSAP6 », on entend notamment des peptides de séquence primaire issue de la séquence primaire de la protéine TSAP6.

La présente invention a bien entendu également pour objet les procédés d'identification des régions des protéines choisies parmi SEQ ID N° 1 à SEQ ID N° 35 ou SEQ ID N° 44 ou 46, ou codées par un acide nucléique choisi parmi SEQ ID N° 36 à SEQ ID N° 43 ou SEQ ID N° 45 ou 47, qui se lient avec TSAP6.

Le système envisagé peut être mis en oeuvre *in vitro*

Ainsi, la présente invention permet l'identification de régions de TSAP6 impliquées dans la liaison avec l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, par un procédé comportant les étapes de :
a) mise en contact de peptides issus de la protéine TSAP6 dans un système permettant la détermination *in vitro* de la liaison entre TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N46 ou 48;
b) identification des peptides entraînant la diminution de la liaison entre TSAP6 et ladite protéine choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 dans ledit système ;

Il est évident que la présente invention permet également la détermination des régions de l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 impliquées dans la liaison avec TSAP6, selon des procédés semblables aux procédés décrits précédemment que ces régions peuvent notamment être utilisées comme leurres lorsque l'on désire diminuer la réversion tumorale et/ou l'apoptose.

La présente invention permet donc d'identifier des produits permettant d'interagir avec la liaison TSAP6 - l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, et donc pouvant avoir un intérêt dans la régulation de l'apoptose et/ou la réversion tumorale. Toutefois, il est possible que ces produits, pour pouvoir être utilisés pour un traitement thérapeutique, notamment le cancer ou les maladies neuro-dégénératives, nécessitent d'être optimisés, afin d'avoir une activité supérieure, et/ou une toxicité moindre.

En effet, le développement d'un médicament est souvent effectué sur le principe suivant :
- criblage de composés ayant une activité voulue par une méthode approprié,
- sélection des composés répondant au « cahier des charges »,
- détermination de la structure (notamment la séquence (éventuellement tertiaire) s'il s'agit de peptides, formule et squelette s'il s'agit de composés chimiques) des composés sélectionnés,
- optimisation des composés sélectionnés, par modification de la structure (par exemple par le changement de la conformation stéroéochimique (par exemple passage de L en D des acides aminés dans un peptide), ajout de substituants sur les squelettes peptidiques ou chimiques, notamment par greffage de résidus sur le squelette, modification des peptides (voir notamment Gante ("Peptidomimetics", dans Angewandte Chemie-International Edition Engl. 1994, 33. 1699-1720),
- passage et criblage des composés ainsi obtenus sur des modèles appropriés qui sont souvent des modèles plus proches de la pathologie étudiée. A ce stade, on utilise notamment souvent des modèles animaux non-humains en général chez les rongeurs (souris, rats...) ou chez des chiens, voire des primates.

Les modèles animaux non-humains qui peuvent être utilisés sont par exemple, pour le cancer, des modèles basés sur des souris immunodéprimées (par exemple *scid*/*scid),* auxquelles on injecte (notamment en sous-cutané) des cellules tumorales, qui vont mener au développement de tumeurs. On étudie l'efficacité des composés potentiellement anti-tumoraux, par exemple par la mesure de la taille des tumeurs formées.

On peut, pour l'étude des maladies neuro-dégénératives, utiliser le modèle décrit par Amson *et al.* (2000, Proc. Natl. Acad. Sci. USA, 97, 5346-50), qui consiste en des souris déficientes en p53, ou le modèle décrit dans Chen *et al.,* Janus *et al.,* et Morgan *et al.* (2000, Nature, 408 pp. 975-985)

Ainsi, la présente invention a notamment pour objet de permettre l'identification de composés qui pourraient être utilisés pour le traitement du cancer, en ce qu'ils possèdent une activité d'augmentation de la réversion tumorale et/ou de l'apoptose. Un des objets de la présente invention est donc un procédé comportant les étapes de :
a) mise en oeuvre d'un procédé selon l'invention, permettant d'identifier des composés ayant une certaine activité d'augmentation de la réversion tumorale et/ou de l'apoptose, et/ou d'inhiber la liaison entre TSAP 6 et une protéine choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48,
b) modification du produit sélectionné à l'étape a),
c) test du produit modifié dans l'étape b) dans des procédés *in vitro* sur des modèles pertinents de réversion tumorale et/ou d'apoptose,
d) identification du produit permettant d'obtenir une activité de réversion tumorale et/ou d'apoptose supérieure à l'activité obtenue pour le produit sélectionné à l'étape a).

L'homme du métier saura définir les conditions et les seuils nécessaires pour l'identification d'un produit pouvant être utilisé en tant que médicament, selon les exigences réglementaires (notamment de toxicologie), par rapport au bénéfice apporté par le produit ainsi identifié.

De la même façon, l'invention concerne également les procédés d'optimisation des produits réprimant la réversion tumorale et/ou l'apoptose, identifiés par les procédés décrits plus haut, et permettant l'identification de produits utilisables comme médicaments.

Ainsi l'invention concerne également un procédé d'identification d'un produit ayant une activité de diminution et/ou d'inhibition de la réversion tumorale et/ou de l'apoptose, caractérisé en ce qu'il comporte les étapes de :
a) mise en oeuvre d'un procédé selon l'invention, permettant d'identifier des composés ayant une certaine activité de diminution de la réversion tumorale et/ou de l'apoptose, et/ou d'inhiber la liaison entre TSAP 6 et une protéine choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48,
b) modification du produit sélectionné à l'étape a) notamment par greffage de résidus sur le squelette chimique,
c) test du produit modifié dans l'étape b) dans des procédés *in vitro* sur des modèles pertinents de réversion tumorale et/ou d'apoptose,
d) identification du produit permettant d'obtenir une activité de réversion tumorale et/ou d'apoptose réduite par rapport à l'activité obtenue pour le produit sélectionné à l'étape a).

Il s'agit en effet encore de pouvoir obtenir le produit qui présente le meilleur rapport (activité biologique et effet clinique) / (risques potentiels d'utilisation).

Les paramètres à mettre en jeu pour obtenir ces résultats sont tous connus et à la portée de l'homme du métier, désirant développer de nouveaux médicaments, et peuvent être trouvés par exemple dans les directives des organismes tels que l'Agence du Médicament, la Commission Européenne, ou la Fédéral Drug Agency.

La mise en oeuvre des procédés selon la présente invention nécessite des modèles permettant de déterminer la liaison entre TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48.

Lorsque l'on met en oeuvre les procédés selon l'invention sur des modèles *in vitro,* pour étudier la liaison entre l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 et TSAP6, il existe plusieurs façons d'opérer.

Un protocole utilisable peut être le suivant :
- expression et purification des protéines TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, par exemple dans des cellules procaryotes (*E. coli, B. subtiis...)* ou eucaryotes (levures telles *Saccharomyces, Kluyveromyces...,* cellules mammifères (HeLa, Cos, Hep-2...) ou d'insectes (en utilisant un système à Baculovirus). Il peut être avantageux que les protéines possèdent une étiquette à leur extrémité N- ou C-terminale, afin de faciliter la purification. On choisit notamment une étiquette Histidine, ou GST. Ces méthodes sont bien connues de l'homme du métier, qui trouve les plasmides appropriés dans les catalogues de sociétés telles Stratagène.
- liaison des protéines à des billes appropriées. Lorsque l'on utilise une étiquette GST, on lie les protéines exprimées à des billes de sépharose présentant de la Gluthathione.
- préparation de protéines par traduction *in vitro.* Ceci peut aisément être effectué en utilisant des vecteurs commerciaux (par exemple disponibles chez Promega), qui permettent de cloner les ADNc sous le contrôle de promoteurs bien connus (T7 ou T3), et d'utiliser les ARN polymérases appropriées pour produire les ARN, puis d'effectuer l'expression des protéines *in vitro.,* en utilisant les trousses disponibles, et en suivant les indications du fabriquant.
- co-précipitation des protéines, en ajoutant les protéines obtenues par traduction *in vitro* aux billes de sépharose - glutathione sur lesquelles sont fixées les protéines de fusion avec la GST. Après un temps de contact suffisant, on lave les billes et on effectue une analyse par gel d'électrophorèse en SDS-PAGE, et autoradiographie. L'apparition des bandes correspondant aux deux protéines montre bien la liaison entre celles-ci. '

L'utilisation de contrôles appropriés permet ainsi de définir la diminution et/ou l'inhibition de la liaison entre TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, par comparaison des quantités de protéines libérées après ajout du composé testé lors de l'étape de coprécipitation avec les quantités de protéines libérées dans les contrôles.

On peut aussi étudier la liaison des protéines en utilisant le système FRET (Fluorescence Resonance Energy Transfer) qui consiste à marquer chacune des protéines avec un résidu approprié, la liaison des deux protéines induisant une réaction entre chacun des deux résidus et l'émission d'une fluorescence aisément détectable.

La présente invention a également pour objet les composés pouvant être obtenu par un procédé selon l'invention, notamment les composés possédant une activité d'augmentation de la réversion tumorale et/ou de l'apoptose, ceux possédant une activité d'inhibition de la liaison TSAP6 - l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, ainsi que ceux possédant une activité de diminution et/ou d'inhibition de la réversion tumorale et/ou de l'apoptose.

La présente invention concerne également les séquences peptidiques correspondant à une région de TSAP6 interagissant avec la protéine l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, qui peuvent notamment être identifiées par un procédé selon l'invention.

L'invention concerne aussi les séquences peptidiques correspondant à une région de l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 interagissant avec la protéine TSAP6, qui peuvent notamment être identifiées par un procédé selon l'invention, le procédé permettant d'identifier les séquences peptidiques de TSAP6 interagissant avec l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 pouvant être adapté pour déterminer les séquences peptidiques de l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 interagissant avec TSAP6, notamment en adaptant le protocole *in vitro* développé ci-dessus.

L'invention concerne aussi les séquences nucléotidiques codant pour les séquences peptidiques ainsi identifiées.

Il est clair que le terme séquence peptidique ou séquence nucléique ou nucléotidique (ces deux derniers termes étant utilisés de façon indifférente) représentent des séquences isolés, c'est-à-dire hors de leur état naturel, et peuvent notamment être modifiés par le remplacement de leurs unités de bases par des unités non-naturelles, ou par la modification des liaisons entre les unités de bases (par exemple les phosphorothioates (acide nucléique) ou les Peptid Nucleic Acids)

La présente invention a donc notamment pour objet de permettre l'identification de composés interférant avec la liaison TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, certains de ces composés pouvant notamment induire des effets sur la cascade de p53. Ainsi, les composés selon l'invention, les séquences peptidiques selon l'invention, ou les séquences nucléotidiques selon l'invention, à titre de médicament, sont également objets de l'invention.

Un composé identifié par une méthode selon l'invention peut être un composé ayant une structure chimique, un lipide, un sucre, une protéine, un peptide, un composé hybride protéine-lipide, protéine-sucre, peptide-lipide, ou peptide-sucre, une protéine ou un peptide sur lequel on a ajouté des ramifications chimiques.

Parmi les composés chimiques envisagés, ils peuvent contenir un ou plusieurs (notamment 2 ou 3) cycles, aromatique(s) ou non, ayant de 3 à 8 atomes de carbones, ainsi que plusieurs résidus de toute sorte (notamment alkyle inférieur, c'est-à-dire présentant entre 1 à 6 atomes de carbones).

Ces composés, séquences nucléiques, séquences peptidiques, peuvent ainsi être utilisés, selon l'invention, pour la préparation d'un médicament, destiné notamment au traitement du cancer, ou d'une maladie neuro-dégénéraxive, selon l'effet pro- ou anti- apoptose/réversion tumorale.

Les inventeurs de la présente demande ont, pour la première fois, mis en évidence le fait que la protéine TSAP6 se lie à la protéine l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48. Ainsi, la présente invention concerne également un complexe constitué d'une protéine TSAP6 et d'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID NI 43 ou SEQ ID NI 46 ou 48.

La présente invention concerne également un procédé d'inhibition in vitro de la liaison de TSAP6 à l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 dans une cellule, comprenant l'étape de :
a) mise en contact de ladite cellule in vitro avec un composé identifié par un procédé selon l'invention, inhibant la liaison TSAP6-l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48.

Le composé ainsi envisagé peut également être un peptide « leurre » issu de la protéine TSAP6 ou de la protéine l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48. Le procédé peut être mis en oeuvre *in vitr*o

### LEGENGE DES FIGURES

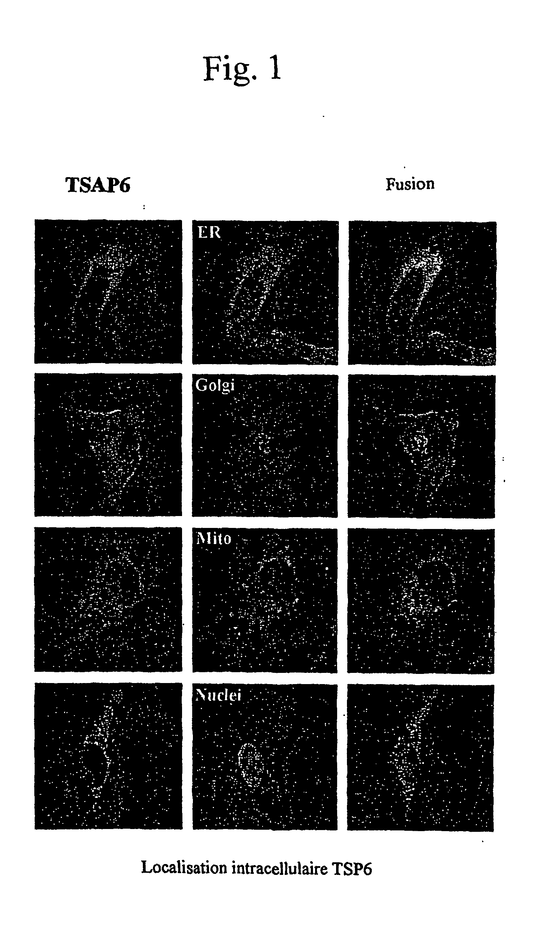

### Figure 1 : TSAP6 colocalise dans le réticulum endoplasmique et golgi

Des cellules Hela transfectées avec GFP-TSAP6 ont été analysées pour leur localisation subcellulaire. Les cellules Hela surexprimant GFP-TSAP6 ont été co-marquées avec des marqueurs spécifiques du réticulum endoplasmique (ER), du golgi, des mitochondries (mito) et du noyau (Nuclei). Ce type de marquage a révélé que la localisation subcellulaire de TSAP6 est à l'intérieur du réticulum endoplasmique et du golgi. Il convient également de noter que TSAP6 apparaît également comme étant exprimé sur les membranes plasmatiques et nucléaires.

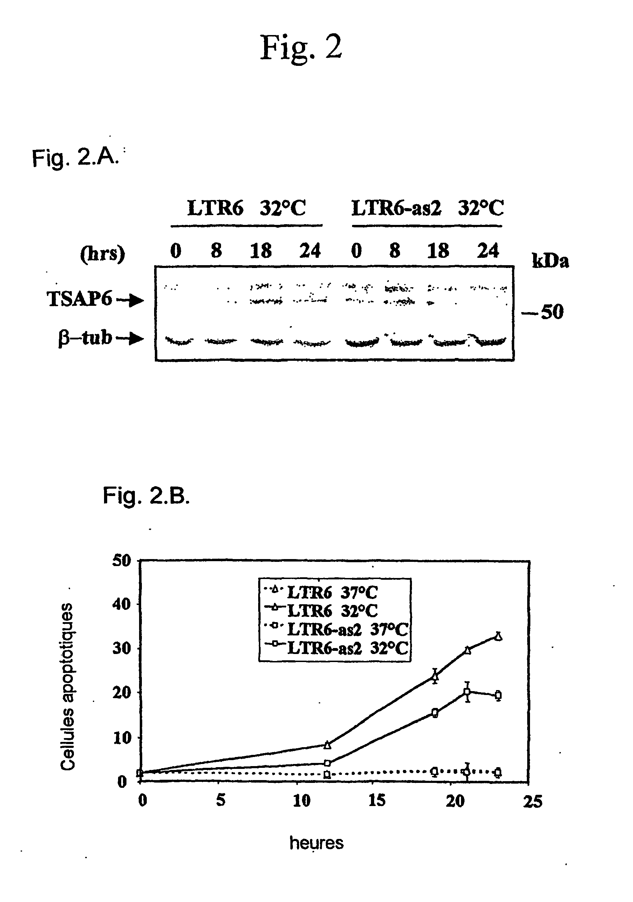

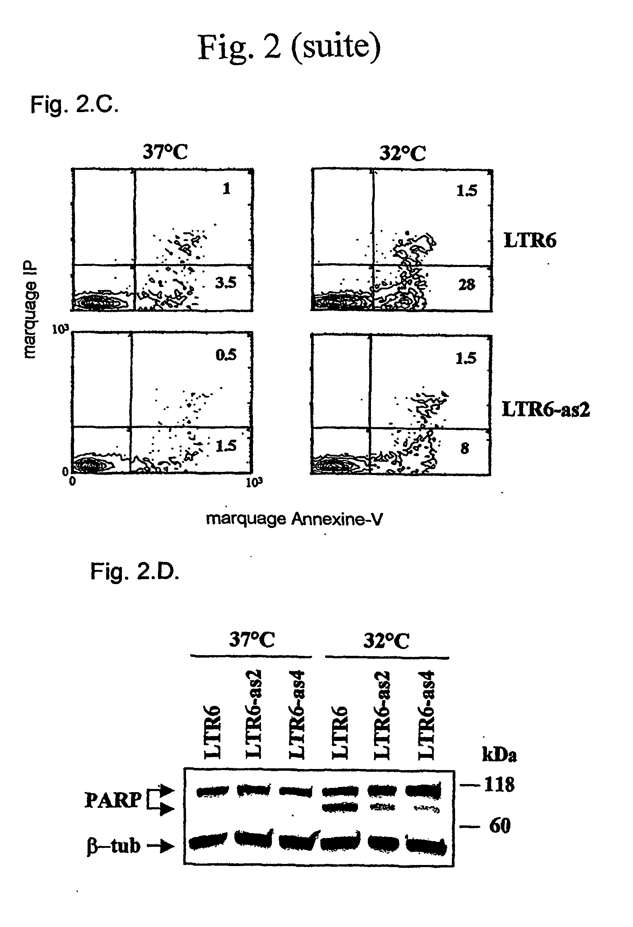

### Figure 2 : L'anti-sens de TSAP6 empêche la mort cellulaire induite par p53 chez les cellules TS LTR6

A. L'analyse par Western blot indique que les niveaux protéiques endogènes de TSAP6 sont diminués 24 h après l'induction par p53 dans les cellules LTR6 surexprimant anti-TSAP6.

B. La surexpression de l'anti-sens TSAP6 (as2) retarde l'apoptose induite par l'activation par p53. L'analyse de l'évolution dans le temps de LTR6 et LTR6-as2 selon la température change à 32°C.

C. Analyse par cytométrie en flux à deux couleurs des cellules LTR6 et LTR6-as2. Des cellules ont été marquées avec de l'annexine-V et de l'iodure de propridium (IP). Les cellules LTR6-as2 expriment moins l'annexine-V et l'annexine-V/IP comparativement aux cellules LTR6, indiquant que l'anti-sens TSAP6 empêche l'apoptose induite par p53.

D. LTR6-as2 et -as4 empêche le clivage PARP induit par p53. Des lysats totaux à partir des expérimentations ci-dessus ont été analysés pour le clivage PARP, un marqueur d'apoptose avec un anticorps contre PARP. Comme comparé aux cellules LTR6, LTR6-as2 et -as4 montrent une réduction marquée de PARP 8 heures après l'activation par p53.

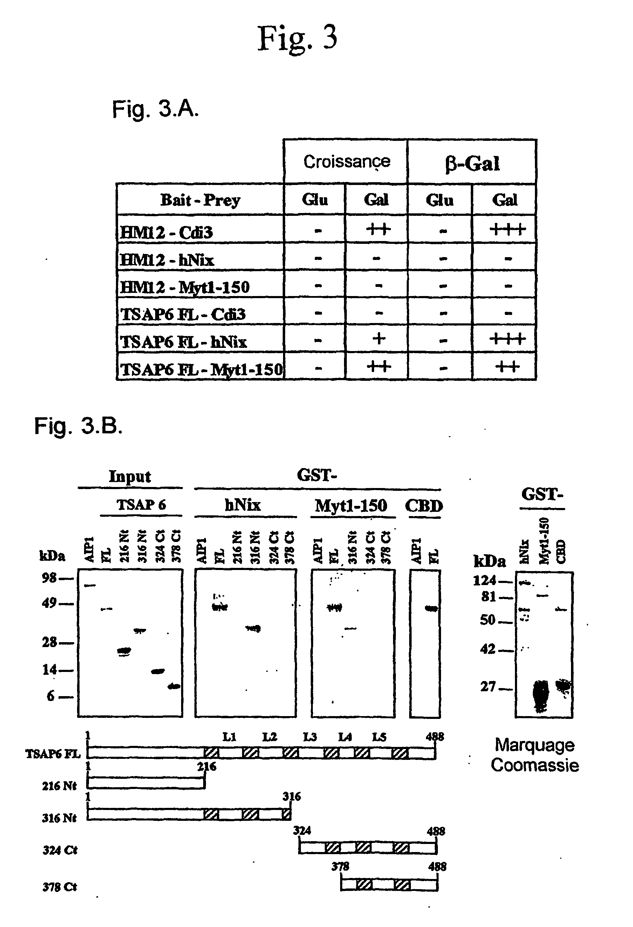

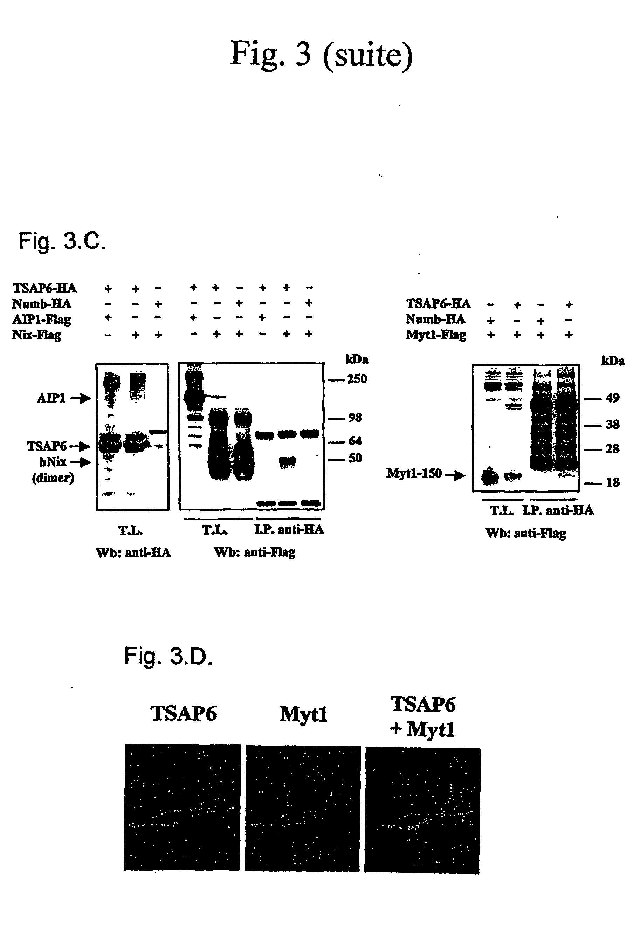

### Figure 3 : TSAP6 se lient avec Nix et Mytl in vitro et in vivo

A. Les analyses d'un tapis de levure ont révélé que TSAP6 interagit avec Nix et Mytl.

B. Interaction *in vitro* de soit GST-Nix, soit GST-Myt1-150 avec TSAP6 radiomarqué lors de la traduction *in vitro* (IVT pour *in vitro* translation). Le GST-Nix ou GST-Mytl-150 est mis à incuber avec du TSAP6 transcrit/traduit radiomarqué *in vitro.* GST-NKTR et AIP1 marqué par IVT sont également inclus en tant que contrôle négatif Ces analyses ont révélé que GST-Nix et GST-Myt1-150 se liaient spécifiquement avec TSAP6. De plus, des expérimentations de cartographie ont révélé que Nix et Myt1 interagissent avec un fragment de TSAP6 composé des acides aminés 1 à 316.

C. Interaction *in vivo* de Ha-TSAP6 avec Flag-Nix (panneau de gauche) et Flag-Myt1-150 (panneau de droite) dans des cellules 293T. Des cellules 293T ont été transfectées avec la combinaison indiquée de plasmides et TSAP6 a été immunoprécipité avec un anticorps anti-HA. Une interaction Nix et Mytl-150 a été révélée avec un anticorps anti-Flag.

D. Colocalisation de GFP-TSAP6 et Flag-Myt1. Des cellules Hela surexprimant GFP-TSAP6 (panneau de gauche) et Flag-Myt1 (panneau du milieu) ont été analysées avec un microscope confocal pour une colocalisation (panneau de droite). Cette analyse indique que TSAP6 et Myt1 sont colocalisés.

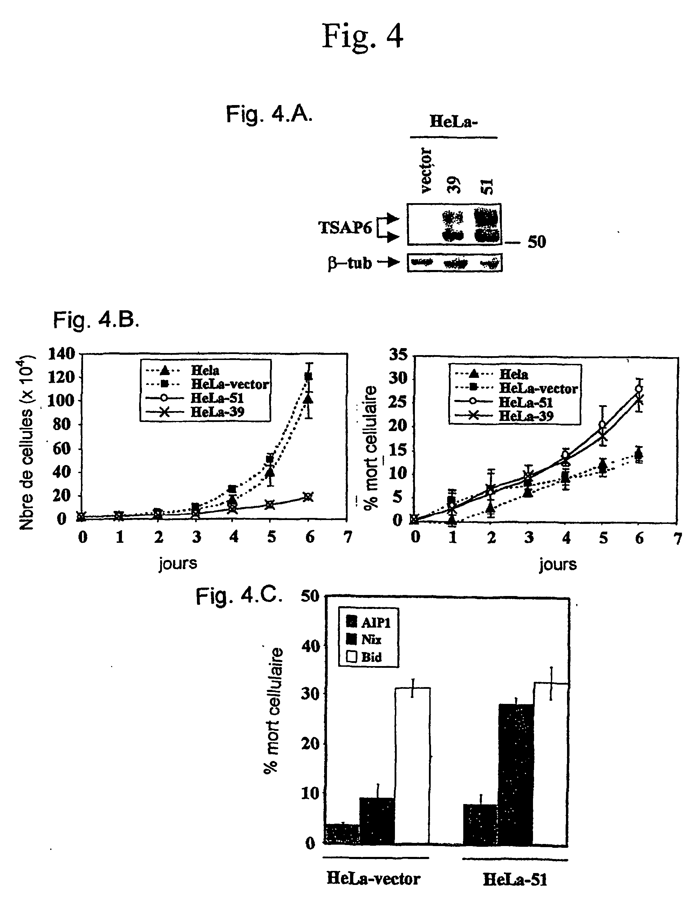

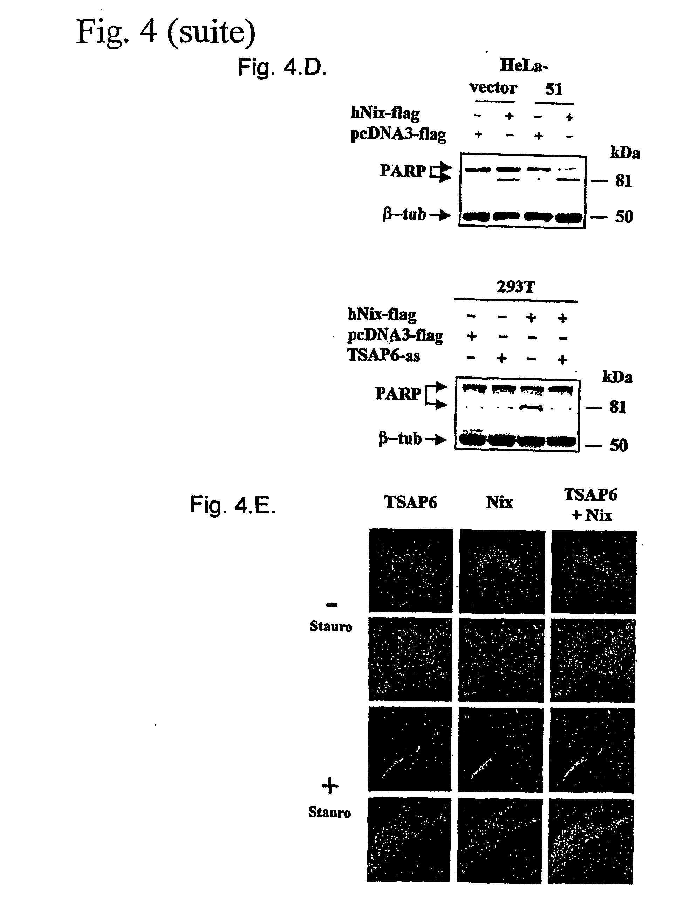

### Figure 4 : TSAP6 coopère avec Nix pour promouvoir la mort cellulaire

A. Cellules Hela 39 et 51 surexprimant HA-TSAP6. Analyse par Western blot utilisant un anti-HA sur des cellules Hela pour détecter TSAP6.

B. La surexpression de TSAP6 favorise le retard de la croissance cellulaire et l'apoptose. Analyse de la croissance cellulaire (panneau de gauche). Les cellules Hela ou les cellules Hela exprimant soit un vecteur contrôle, soit TSAP6 (39 et 51) ont été marquées avec du bleu trypan et la viabilité cellulaire a été déterminée aux temps indiqués. Analyse de la mort cellulaire (panneau de droite) : la mort cellulaire a été déterminée par comptage cellulaire qui incorporait le bleu trypan.

C. Exacerbation de l'apoptose chez les cellules Hela-51 surexprimant Nix. Un vecteur Hela ou des cellules Hela-51 ont été transfectés avec des constructions Flag contenant le contrôle négatif AIP1, Nix ou la molécule pro-apoptotique, Bid-t qui a été utilisée en tant que contrôle positif. 24 heures après la transfection, les cellules ont été marquées avec un anticorps anti-flag pour détecter les protéines surexprimées et avec DAPI, un colorant nucléaire, et ensuite les cellules ont été comptées. Cette analyse a montré que tandis qu'AIP1 avait un effet minimal sur l'apoptose dans une quelconque lignée cellulaire, la surexpression de Nix stimulait fortement la mort cellulaire chez les cellules surexprimant TSAP6. Bid-t, d'un autre côté, favorisait des niveaux similaires d'apoptose indépendamment de la lignée cellulaire.

D. La surexpression de Nix chez Hela-51 favorise fortement le clivage de PARP. Des lysats totaux à partir des expérimentations précédentes ont été analysés par Western blot en utilisant un anticorps anti-PARP. Cette analyse montre que Nix et TSAP6 coopère ensemble pour favoriser des niveaux de clivage de PARP. (ci-dessous) L'anti-sens TSAP6 empêche le clivage de PARP induit par Nix chez les cellules 293T. Des cellules 293T ont été transfectées avec les plasmides indiqués et 24 heures après, des lysats cellulaires ont été préparés et le clivage de PARP a été analysé par immunomarquage avec un anticorps anti-PARP. Ces données montrent que tandis que Nix seul favorise le clivage partiel de PARP, l'addition d'un anti-sens de TSAP6 bloque complètement cet effet.

E. Colocalisation de GFP-TSAP6 et Nix après le traitement à la staurosporine. Des cellules Hela transfectées avec TSAP6 (panneau de gauche) et Nix (panneau du milieu) ont été analysées par microscope confocal 24 heures plus tard. Tandis qu'une colocalisation de TSAP6 et Nix (panneau de droite) a été difficilement détectable (-stauro), l'induction de l'apoptose par la staurosporine a favorisé une coalescence de TSAP6 et Nix.

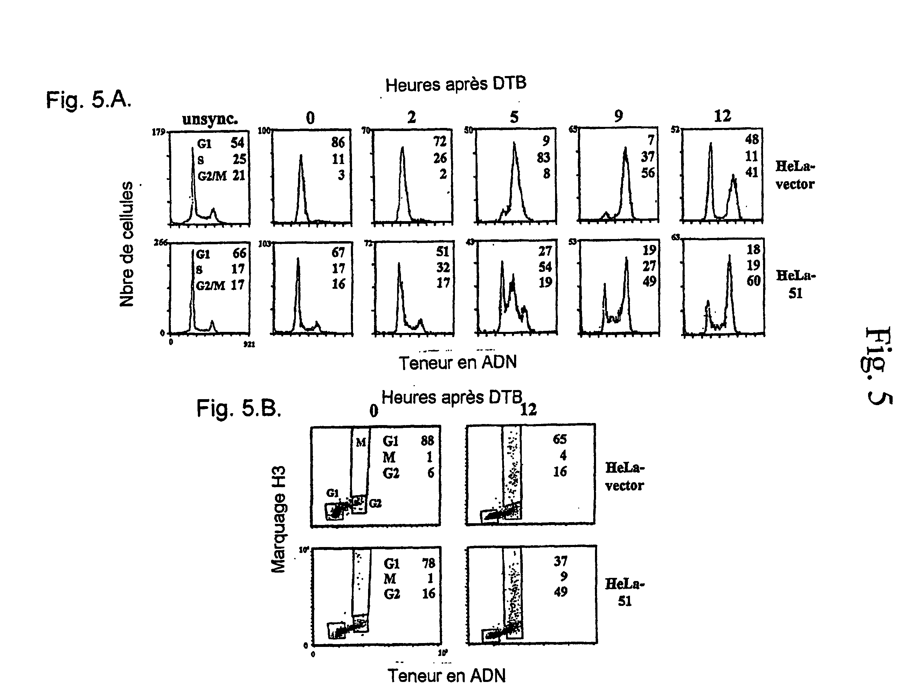

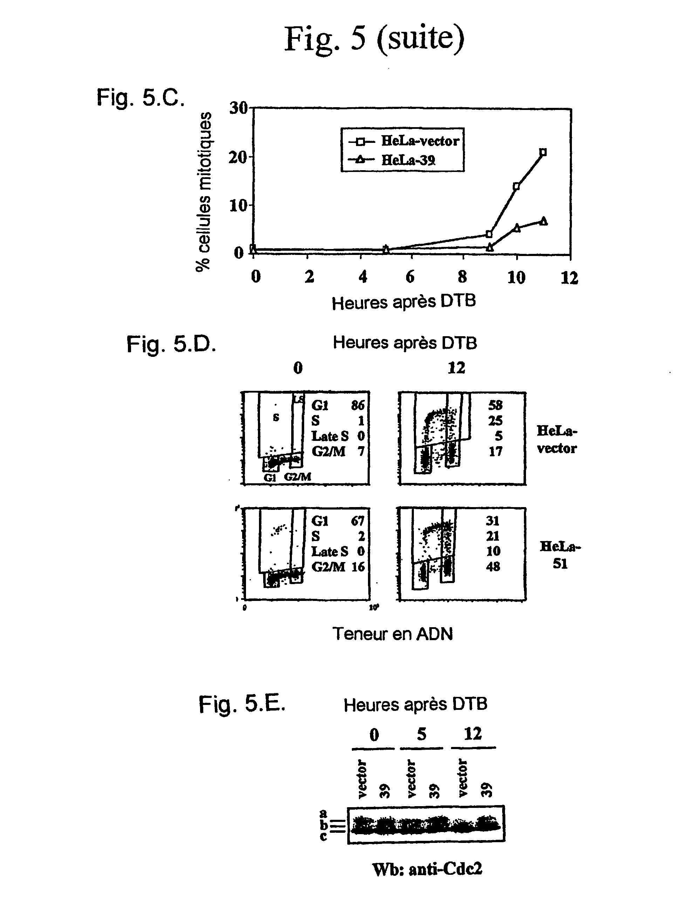

### Figure 5 : La surexpression de TSAP6 favorise le retard du cycle cellulaire en G2 en empêchant la déphosphoryation de cdc2

A. L'analyse par blocage à la thymidine double révèle que les cellules Hela-51 contiennent une population G2/M additionnelle. Une procédure DTB a été réalisée et un vecteur Hela et des cellules Hela-51 ont été analysés aux temps indiqués concernant leur progression dans le cycle cellulaire. Une analyse par cytométrie en flux a été réalisée sur des cellules marquées avec l'iodure de propidium dans le but de révéler les différentes étapes du cycle cellulaire. A t=0 les cellules Hela-51 montraient une population additionnelle de cellules correspondants à G2/M. A t=12 heures, les cellules Hela-51 contenaient une accumulation notable de ces cellules G2/M, à un temps où la majorité des cellules Hela-vecteur retournent au stade G1.

B. Marquage des histone H3 à t=0 et t=12 heures après le relarguage de DTB. L'analyse par cytométrie en flux a été réalisée sur des cellules marquées avec un anticorps phosphohistone anti-H3, lequel marque les cellules en mitose et avec de l'iodure de propidium. Cette analyse a révélé que, tandis que la majorité des cellules Hela-51 présentaient un phénotype G2/M (environ 60%) après 12 heures, seulement environ 15% des cellules G2/M présentaient un phénotype H3⁺. Ceci est en contraste avec les cellules vecteur Hela contenant G2/M dont 20% sont en mitose au même moment.

C. Analyse de l'index mitotique des cellules vecteur-Hela et Hela-51. Ces résultats ont révélé que les cellules Hela-51 ont une entrée retardée en mitose comparativement aux cellules vecteur Hela. De plus, ces données confirment les résultats ci-dessus selon lesquels la surexpression de TSAP6 retarde plutôt qu'il ne favorise la progression du cycle cellulaire. Les cellules Hela ont été synchronisées avec le DTB et aux temps indiqués, les cellules ont été fixées et marquées avec du colorant Giemsa-Wright dans le but de détecter les condensations chromosomiques. Au moins 600 noyaux ont été comptés pour chaque point de temps.

D. Marquage au Brdu des cellules vecteur-Hela et des cellules Hela-51. Les cellules Hela ont été marquées par du Brdu qui détecte les cellules contenant les cellules en phase S et avec de l'iodure de propridium. L'analyse par cytométrie en flux sur les marquages à la Brdu a été réalisée. Ces résultats montrent que les cellules en phase S tardives représentent un petit pourcentage des cellules en phase G2/M pour les deux types cellulaires que sont vecteur-Hela ou Hela-51.

E. Cdc2 est hyperphosphorylé dans les cellules surexprimant TSAP6. Des lysats cellulaires ont été générés aux points de temps indiqués après le relarguage de DTB et l'analyse par immunomarquage a été réalisée en utilisant un anticorps anti-cdc2 de façon à détecter les différentes formes phosphorylées de cdc2. Cette analyse a montré que 12 heures après le relarguage DTB, la majorité de cdc2 est déphosphorylée dans les cellules Hela-vecteur indiquant une forme active. De manière surprenante, par contraste, cdc2 dans les cellules Hela-51 apparaît hyperphosphorylé au même moment.

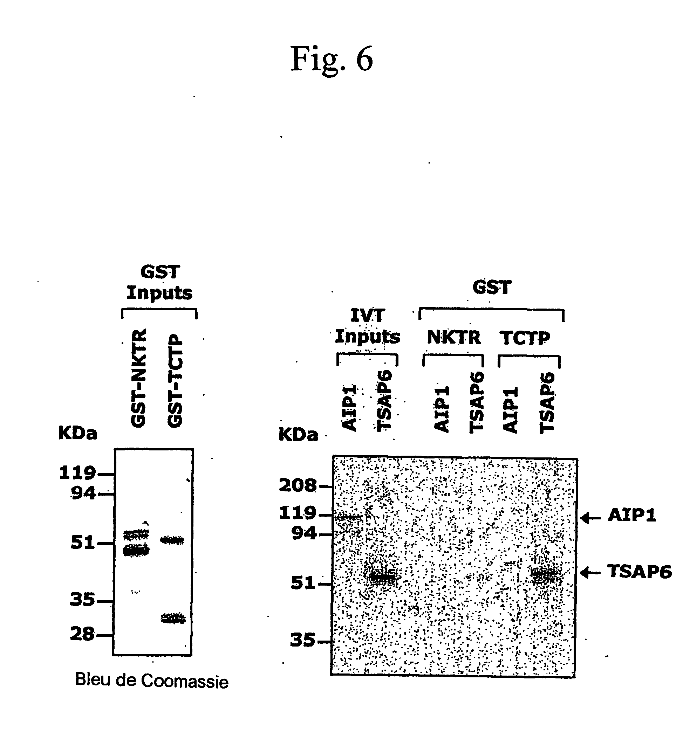

### Figure 6 : GST-TCTP interagit avec TSAP6 in vitro.

Interaction de GST-TCTP et de TSAP6 radiomarqué. TSAP6 et le contrôle négatif AIP1 ont été générés par une traduction *in vitro* (IVT) dans un lysat de réticulocyte de lapin en présence de méthionine et de cystéine marquées au ³⁵S. Des quantités égales de produits radiomarqués ont été incubées avec soit GST-TCTP, soit des protéines de fusion GST-NKTR capturées sur les billes de glutathion en tant que contrôle négatif. Les protéines radiomarquées ont été éluées dans un tampon d'échantillon protéique, résolu dans des conditions de réduction (0,7 mM de 2-β-mercaptoéthanol) par électrophorèse en gel de sodium dodécyl sulfate-polyacrylamide (SDS-PAGE) (10%) et visualisé par autoradiographie.

### EXEMPLES

### Exemple 1 : liaison entre TSAP6 et les protéines choisies parmi SEQ ID N° 1 à SEQ ID N° 35 ou SEQ ID N° 44 ou 46, ou codées par un acide nucléique choisi parmi SEQ ID N° 36 à SEQ ID N° 43 ou SEQ* ID N° 45 ou 47

La liaison existant entre TSAP 6 et chaque protéine choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 a été observée dans un système double-hybride, dérivé du système développé par Finley et Brent *(Interaction trap cloning with yeast,* 169-203, *in* DNA Cloning, Expression Systems : a practical Approach, 1995, Oxford Universal Press, Oxford), en utilisant TSAP 6 en tant qu'appât, et une banque d'ADNc en tant que proie.

La protéine TSAP6 a été clonée dans le plasmide pEG202 connu de l'homme du métier pour une telle application (promoteur 67-1511, lexA 1538-2227, ADH Ter 2209-2522, pBR remnants 2540-2889, 2µ ori 2890-4785, YSCNFLP 4923-5729, HIS3 7190-5699, TYIB 7243-7707, RAF_part 7635-7976, squelette pBR 7995-10166, bla 8131-8988).

Les ADNc de la banque sont clonés dans le plasmide pJG4-5, également bien connu de l'homme du métier (promoteur GAL 1-528, cassette de fusion 528-849, ADH Ter 867-1315, 2µ ori 1371-3365, TRP1 3365-4250, squelette pUC 4264-6422, Ap 4412-5274).

On utilise également le plasmide rapporteur pSH18-34, également connu de l'homme du métier. Ce plasmide est notamment disponible chez Invitrogen, sous le numéro de référence V611-20, et également déjà transformé dans la souche EGY48 (aussi appelée RFY 231), chez le même fournisseur (référence souche seule : C835-00, transformée par pSH18-34 : C836-00)

La liaison a été démontrée dans la souche de levure RFY 231 (décrite dans Finley Jr, *et al,* 1998, Proc Natl Acad Sci USA, 95, 14266-71). Cette souche de levure possède le génotype (MATα *trplΔ::hisG his3 ura3-1 leu2::3Lexop-LEU2),* et est dérivée de EGY48 (Guris *et al.,* 1993, Cell, 75, 791-803).

Le gène rapporteur était le gène LacZ.

On effectue l'étude sur un milieu contenant du galactose, ne contenant pas de leucine, et on étudie la présence de colonies colorées sur ces boîtes.

On peut ainsi montrer la liaison, dans ce système, entre TSAP6 et les protéines choisies parmi SEQ ID N° 1 à SEQ ID N° 35 ou SEQ ID N° 44 ou 46, ou codées par un acide nucléique choisi parmi SEQ ID N° 36 à SEQ ID N° 43 ou SEQ ID N° 45 ou 47.

### Exemple 2 : protocole pour l'expression des protéines de fusion GST

Pour obtenir des protéines de fusion GST, on peut suivre le protocole suivant :
Préparation d'une préculture à partir d'une colonie isolée de BL21 (DE3), transformée avec le plasmide pGEX-6P-1 ou pGEX-P-1, dans un milieu SB avec 100µg/ml d'ampicilline, à 37°C.
Les plasmides sont disponibles chez Amersham Pharmacia Biotech AB.
Les protéines sont les protéines humaines, codées par les ADNc complémentaires (SEQ ID N° 1 à SEQ ID N°7).

Le lendemain, inoculer 250 ml de SB+Amp avec 5 ml de la préculture.

Croissance à 28°C ou 37°C selon la toxicité des protéines pour les bactéries hôtes, jusqu'à une densité optique entre 0,5 et 0,7.
Ajout de 0,1 mM IPTG pour induire la synthèse des protéines.
Croissance à 28°C ou 37°C pendant 1h ou 1h30.
Centrifugation 3000 rpm pendant 10 min (1800g, 4°C)
Resuspension du précipité dans 10 ml de tampon A NP40 (NP40 1 % ; Tris pH 7,4 10 mM ; NaCl 150 mM; EDTA 1 mM ; Glycerol 10%; DTT 1mM; Aprotinin 2µg/ml ; Leupeptin 2µg/ml ; Pepstatin 2µg/ml ; AEBSF 1mM).
Sonication 3 fois pendant 15 s à puissance 50, sur glace.
Centrifugation à 12000 rpm pendant 10 min (18000g, 4°C)
Garder le surnageant à -80°C.

### Exemple 3 : protocole à suivre pour la liaison des protéines de fusion aux billes de sepharose-glutathione

Ajout de 2 ml de surnageant à 200 µl de billes (préparées après 3 rinçages dans le PBS, 1 rinçage dans le tampon A NP40, resuspension à 50 % (poids par volume) dans le tampon A NP40, centrifugation à 3000 rpm à chaque fois).

Les billes Glutathione-sepharose 4B sont disponibles chez Amersham Pharmacia Biotech AB, sous le numéro 17.0756.01.

Remuer doucement pendant au moins 1 heure à 4°C.

Rincer les billes 3 fois dans le tampon A NP40 sans inhibiteur de protéase.

Resuspendre les billes dans 1 ml de tampon A NP40 avec inhibiteur de protéase.

Pour l'analyse par électrophorèse SDS-PAGE, prendre 20 à 40 µl des billes resuspendues, centrifuger pendant 5 min, écarter le surnageant, resuspendre les billes dans 10 µl de tampon de charge de X, chauffer à 97°C pendant 7 min. Charger sur gel et analyser après révélation par bleu de Coomassie, pour normaliser la quantité des protéines de fusion à utiliser.

### Exemple 4 : protocole pour la traduction de protéines in vitro

Le kit TNT Coupled Reticulocyte Lysate System de Promega est utilisé avec les ARN polymérase T7 ou T3 en fonction du vecteur utilisé pour traduire et exprimer les protéines (par exemple T7 pour TSAP6 1, T3 pour la protéine choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48). Le kit est utilisé selon les instructions du fabricant (Référence L4610)

Les protéines incorporent de la méthionine S³⁵ (Amersham Pharmacia).

Les produits obtenus in vitro sont analysés par électrophorèse SDS-PAGE.

Après électrophorèse, le gel est placé dans du tampon de fixation (5 % de méthanol, 15 % d'acide acétique, 80 % d'eau) pendant une 1/2 heure et le signal est amplifié par immersion du gel dans le produit Amplify de Amersham Pharmacia (Ref: NAMP100).

Un film Kodak Biomax MR est alors exposé sur le gel séché pendant une durée variant d'une heure à une semaine, puis développé.

### Exemple 5 : protocole pour la co-précipitation des protéines

30 µl des billes de sépharose- glutathione couplés aux protéines de fusion GST, après normalisation des quantités, sont rincés dans le tampon B (NP40 1 %, TrisHCl 50 mM, NaC1150 mM, leupeptine 2 µl/ml, aprotinine 1 %, ABESF 1 mM). On ajoute ensuite 5 à 10 µl du produit de traduction *in vitro* tel qu'obtenu dans l'exemple 3, en fonction de la quantité du produit observé par autoradiographie.

Après une nuit de contact, les billes sont rincées 10 fois avec du tampon A NP 40, sans antiprotéases.

On analyse par SDS-PAGE et autoradiographie.

On peut ainsi montrer la liaison entre les protéines TSAP6 et ladite protéine choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48.

### Exemple 6 : protocole pour le criblage de composés interférant avec la liaison TSAP6 - l'une des protéines parmi SEQ ID N° 1 à SEQ ID N° 35 ou SEQ ID N° 44.45 ou 47, ou codée par un acide nucléique choisi parmi SEQ ID N° 36 à SEQ ID N° 43 ou SEQ ID N° 46 ou 48

On effectue les essais décrits dans les exemples 2 à 5, en ajoutant les composés que l'on désire étudier à l'étape de l'exemple 5, et on compare avec les résultats obtenus lorsque les composés ne sont pas ajoutés.

### MATERIELS ET METHODES

### Expression et purification des protéines de fusion GST

Une pré-culture de TCTP pGEX-6P-1 ou de NKTR dans SB+Ampicilline 100 µg/ml à partir d'une simple colonie a été préparée pendant toute la nuit, à 37°C.

Au matin, 250 ml de LB+Amp avec 5 ml de cette pré-culture ont été inoculés.

Croissance à 37°C jusqu'à ce que la DO atteigne 0,5 à 1 (environ 3 heures).
Addition de 0,1 mM d'IPTG.
Croissance pendant encore 1 h 30.
Centrifugation à 3 000 rpm pendant 10 mn.

Resuspension du culot dans 10 d'un tampon A de lyse NP40, de façon à éviter des bulles :
NP40 1%*
Tris pH 7,4 10 mM^{*}
NaCl 150 mM*
EDTA 1 mM^*
Glycérol 10 %*
DTT 1 mM
Aprotinine 2 µg/ml
Leupeptine 2 µg/ml
Pepstatine 2 µg/ml
AEBSF 1 mM

Sonication 3 fois 15 secondes dans la glace toutes les 15 secondes à une puisse de 40.

Centrifugation à 12 000 rpm = 18 000 g (centrifugeuse 1510R) à 4°C pendant 30 mn.

Conservation avec précaution du surnageant à - 80°C si nécessaire.

### Liaison aux billes d'agarose-glutathion

Addition de 2 ml de surnageant à 200 µl de billes (elles doivent être préparées avec 3 rinçages au PBS, 1 rinçage avec le tampon NP40 et des inhibiteures de protéase, resuspendu à 50 % dans ce tampon NP40, centrifugation à 1 500 rpm = 900 g (centrifugeuse 1510R) à 4°C, chaque fois).

Agitation douce pendant au moins 1 heure à 4°C.

Rinçage des billes 3 fois dans du tampon A NP40 sans inhibiteur de protéase dans 10 ml de tampon NP40 chaque fois (50 volumes).

Resuspension de 200 µl de billes dans 200 µl de tampon NP40 avec des inhibiteurs de protéase pour obtenir une solution à 50 %.

Analyse du gel : centrifugation de 20 µl pendant 5 minutes, élimination du surnageant, resuspension des billes dans 10 µl 2 fois dans un tampon échantillon, chauffage à 97°C pendant 7 mn. Chargement du gel pour normalisation d'une quantité de protéines de fusion GST. Des gels Novex NuPAGE à 10 % ont été utilisés avec du tampon MES pour obtenir une bonne résolution.

### Traduction in vitro

Le kit « TNT Coupled Reticulocyte Lysate System » de PROMEGA a été utilisé avec la polymérase T7. Le protocole régulier a été suivi.

Les protéines ont été radiomarquées avec de la méthionine ³⁵S ou de la méthionine ³⁵S, cystéine d'Amersham Pharmacia.

| | | | | |
|---|---|---|---|---|
| Volumes de réactions totales | 50 µl | 100 µl | 150 µl | 200 µm |
| Lysat rouge (µl) | 25 | 50 | 75 | 100 |
| Tampon TNT(µl) | 2 | 4 | 6 | 8 |
| AA mix-Met ou Met et -Cys (µl) | 2 | 4 | 6 | 8 |
| RNAsine (µl) | 1 | 2 | 3 | 4 |
| T7 RNA polymérase (µl) | 1 | 2 | 3 | 4 |
| 35S Met ou 35S Met, Cys (µl) | 3 | 5 | 7 | 9 |
| H20(µl) | 15 | 32 | 49 | 66 |
| ADN pl | 0,5 µg | 1 µg | 1,5 µg | 2 µg |

Le mélange a été préparé dans la glace. La radioactivité a été ajoutée à la fin.
La réaction s'est déroulée à 30°C pendant 1 h 30.

Pendant la traduction *in vitro,* les billes ont été préparées : calcul de la quantité de chaque protéine de fusion liée à des billes d'agarose-glutathion. Dans chaque cas, addition de billes pleines pour avoir un volume total de 30 µl de billes par interaction, dans le but d'être capable de les voir au fond du tube. Les billes ont été rincées 3 fois dans 1 ml de tampon B : NP40 1 %, TrisHCl 50 mM, NaCl 150 mM, Leupeptine 2 µg/ml, Aprotinine 1 %, AEBSF 1 mM.

Un aliquot de 30 µl de billes (60 µl d'une solution à 50 %) a été placé dans des tubes Eppendorf. Addition de 50 µl de tampon B NP40 + des inhibiteurs dans chaque tube pour avoir un volume conséquent durant l'interaction. Les contrôles positifs et négatifs n'ont pas été oubliés. Il convient d'éviter la GST seule en tant que contrôle négatif car c'est trop visqueux.

Dès que la traduction *in vitro* a eu lieu, addition de 3 à 30 µl d'IVT (selon la normalisation recherchée) à chaque 30 µl de billes).

Addition de SB 2X contenant 0,7 mM de beta-mercapto éthanol au reste d'IVT pour analyser le gel. Chauffage des échantillons à 80°C pendant 5 mn avant de charger.

Après une nuit de contact à 4°C ou 1 heure à température ambiante, les billes sont rincées 10 fois dans du tampon A ou B (si une stringence importante est acceptable) (10X 1,5 ml) à 4°C. Après le rinçage, les billes sont recouvertes avec 10 ml de tampon auquel on additionne 30 µl de 2 fois SB contenant 0,7 mM de beta-mercapto éthanol, chauffé à 80°C pendant 5 mn avant l'analyse par SDS-PAGE. 12 à 20 µl des échantillons peuvent être chargés dans chaque puits. Après l'électrophorèse, le gel est mis à tremper dans un tampon de fixation (5 % de méthanol, 15 % d'acide acétique, 80 % d'eau) pendant 30 mn et le signal est amplifié par immersion du gel dans le produit Amplify d'Amersham Pharmacia.

Un film Kodak Biomax MR est ensuite exposé sur le gel sec pendant 1 heure à 1 semaine et développé.

## Revendications

1. Procédé d'identification d'un composé inhibant la liaison de TSAP6 à l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, présentant les étapes de :
a) mise en contact dudit composé avec un système permettant la détermination *in vitro* de la liaison entre TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 ;
b) identification de la diminution et/ou l'inhibition de la liaison entre TSAP6 et ladite protéine définie en a).

2. Procédé d'identification d'un composé permettant l'augmentation de la réversion tumorale et/ou la mort cellulaire (apoptose), **caractérisé en ce qu'**il présente les étapes de :
a) mise en contact de composés avec un système permettant la détermination *in vitro* de la liaison entre TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 ;
b) identification des composés induisant la diminution et/ou l'inhibition de la liaison entre TSAP6 et ladite protéine définie en a) ;
c) mise en contact des composés identifiés dans l'étape b) dans un système in vitro permettant de mesurer les phénomènes d'apoptose et/ou de réversion tumorale ;
d) identification de l'augmentation de la réversion tumorale et/ou la mort cellulaire (apoptose) dans ledit modèle par rapport à un modèle témoin avec lequel ledit composé n'a pas été mis en contact.

3. Procédé d'identification d'un composé pour la diminution et/ou l'inhibition de la réversion tumorale et/ou la mort cellulaire (apoptose), présentant les étapes de :
a) mise en contact de composés avec un système permettant la détermination *in vitro* de la liaison entre TSAP6 et l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 ;
b) identification des composés induisant la diminution et/ou l'inhibition de la liaison entre TSAP6 et ladite protéine définie en a);
c) mise en contact des composés identifiés dans l'étape b) dans un système in vitro permettant de mesurer les phénomènes d'apoptose et/ou de réversion tumorale ;
d) identification de la diminution et/ou l'inhibition de la réversion tumorale et/ou la mort cellulaire (apoptose) dans ledit modèle par rapport à un modèle témoin avec lequel ledit composé n'a pas été mis en contact.

4. Procédé d'identification des régions de TSAP6 impliquées dans la liaison avec l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48, comportant les étapes de :
a) mise en contact de peptides issus de la protéine TSAP6 dans un système permettant la détermination *in vitro* de la liaison entre TSAP6 et ladite protéine choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 ;
b) identification des peptides entraînant la diminution de la liaison entre TSAP6 et ladite protéine choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48 dans ledit système ;

5. Procédé d'identification d'un produit ayant une activité d'augmentation de la réversion tumorale et/ou de l'apoptose, **caractérisé en ce qu'**il comporte les étapes de :
a) mise en oeuvre d'un procédé selon l'une des revendications 1 ou 2,
b) modification du produit sélectionné à l'étape a) notamment par greffage de résidus sur le squelette chimique,
c) test du produit modifié dans l'étape b) dans des procédés *in vitro* sur des modèles pertinents de réversion tumorale et/ou d'apoptose,
d) identification du produit permettant d'obtenir une activité de réversion tumorale et/ou d'apoptose supérieure à l'activité obtenue pour le produit sélectionné à l'étape a).

6. Procédé d'identification d'un produit ayant une activité de diminution et/ou d'inhibition de la réversion tumorale et/ou de l'apoptose, **caractérisé en ce qu'**il comporte les étapes de :
a) mise en oeuvre d'un procédé selon l'une des revendications 1 ou 3,
b) modification du produit sélectionné à l'étape a) notamment par greffage de résidus sur le squelette chimique,
c) test du produit modifié dans l'étape b) dans des procédés *in vitro* sur des modèles pertinents de réversion tumorale et/ou d'apoptose,
d) identification du produit permettant d'obtenir une activité de réversion tumorale et/ou d'apoptose réduite par rapport à l'activité obtenue pour le produit sélectionné à l'étape a).

7. Complexe constitué d'une protéine TSAP6 et de l'une des protéines choisie parmi SEQ ID N° 1 à SEQ ID N° 35 ou SEQ ID N° 44, 45 ou 47, ou codée par un acide nucléique choisi parmi SEQ ID N° 36 à SEQ ID N° 43 ou SEQ ID N° 46 ou 48.

## Claims

1. Method for identifying a compound which inhibits the binding of TSAP6 to one of the proteins chosen from SEQ ID No 1 to SEQ ID No 35 or SEQ ID No 44, 45 or 47, or encoded by a nucleic acid chosen from SEQ ID No 36 to SEQ ID No 43 or SEQ ID No 46 or 48, having the steps of:
a) bringing said compound into contact with a system for determining, *in vitro,* the binding between TSAP6 and one of the proteins chosen from SEQ ID No 1 to SEQ ID No 35 or SEQ ID No 44, 45 or 47 or encoded by a nucleic acid chosen from SEQ ID No 36 to SEQ ID No 43 or SEQ ID No 46 or 48;
b) identifying the decrease in and/or the inhibition of the binding between TSAP6 and said protein defined in a).

2. Method for identifying a compound which makes it possible to increase tumour reversion and/or cell death (apoptosis), **characterized in that** it has the steps of:
a) bringing compounds into contact with a system for determining, *in vitro,* the binding between TSAP6 and one of the proteins chosen from SEQ ID No 1 to SEQ ID No 35 or SEQ ID No 44, 45 or 47, or encoded by a nucleic acid chosen from SEQ ID No 36 to SEQ ID No 43 or SEQ ID No 46 or 48;
b) identifying the compounds which induce the decrease in and/or the inhibition of the binding between TSAP6 and said protein defined in a);
c) bringing the compounds identified in step b) into contact in an in vitro system for measuring the phenomena of apoptosis and/or of tumour reversion;
d) identifying the increase in tumour reversion and/or in cell death (apoptosis) in said model by comparison with a control model with which said compound has not been brought into contact.

3. Method for identifying a compound for decreasing and/or inhibiting tumour reversion and/or cell death (apoptosis), having the.steps of:
a) bringing compounds into contact with a system for determining, *in vitro,* the binding between TSAP6 and one of the proteins chosen from SEQ ID No 1 to SEQ ID No 35 or SEQ ID No 44, 45 or 47, or encoded by a nucleic acid chosen from SEQ ID No 36 to SEQ ID No 43 or SEQ ID No 46 or 48;
b) identifying the compounds which induce the decrease in and/or the inhibition of the binding between TSAP6 and said protein defined in a);
c) bringing the compounds identified in step b) into contact in an in vitro system for measuring the phenomena of apoptosis and/or of tumour reversion;
d) identifying the decrease in and/or the inhibition of tumour reversion and/or cell death (apoptosis) in said model by comparison with a control model with which said compound has not been brought into contact.

4. Method for identifying regions of TSAP6 which are involved in the binding with one of the proteins chosen from SEQ ID No 1 to SEQ ID No 35 or SEQ ID No 44, 45 or 47, or encoded by a nucleic acid chosen from SEQ ID No 36 to SEQ ID No 43 or SEQ ID No 46 or 48, comprising the steps of:
a) bringing peptides derived from the TSAP6 protein into contact in a system for determining, *in vitro,* the binding between TSAP6 and said protein chosen from SEQ ID No 1 to SEQ ID No 35 or SEQ ID No 44, 45 or 47, or encoded by a nucleic acid chosen from SEQ ID No 36 to SEQ ID No 43 or SEQ ID No 46 or 48;
b) identifying the peptides which lead to the decrease in the binding between TSAP6 and said protein chosen from SEQ ID No 1 to SEQ ID No 35 or SEQ ID No 44, 45 or 47, or encoded by a nucleic acid chosen from SEQ ID No 36 to SEQ ID No 43 or SEQ ID No 46 or 48, in said system.

5. Method for identifying a product having an activity of increasing tumour reversion and/or apoptosis, **characterized in that** it comprises the steps of:
a) implementing a method according to either of Claims 1 and 2,
b) modifying the product selected in step a), in particular by grafting residues onto the chemical backbone,
c) testing the product modified in step b) in in vitro methods on relevant models of tumour reversion and/or apoptosis,
d) identifying the product which makes it possible to obtain an activity of tumour reversion and/or apoptosis greater than the activity obtained for the product selected in step a).

6. Method for identifying a product having an activity of decreasing and/or inhibiting tumour reversion and/or apoptosis, **characterized in that** it comprises the steps of:
a) implementing a method according to either of Claims 1 and 3,
b) modifying the product selected in step a), in particular by grafting residues onto the chemical backbone,
c) testing the product modified in step b) in in vitro methods on relevant models of tumour reversion and/or apoptosis,
d) identifying the product which makes it possible to obtain an activity of tumour reversion and/or apoptosis which is decreased compared to the activity obtained for the product selected in step a).

7. Complex consisting of a TSAP6 protein and of one of the proteins chosen from SEQ ID No 1 to SEQ ID No 35 or SEQ ID No 44, 45 or 47, or encoded by a nucleic acid chosen from SEQ ID No 36 to SEQ ID No 43 or SEQ ID No 46 or 48.

## Patentansprüche

1. Verfahren zur Identifizierung einer Verbindung, welche die Bindung von TSAP6 an eines der Proteine, welches aus SEQ ID Nr. 1 bis SEQ ID Nr. 35 oder SEQ ID Nr. 44, 45 oder 47 ausgewählt oder durch eine Nukleinsäure, ausgewählt aus SEQ ID Nr. 36 bis SEQ ID Nr. 43 oder SEQ ID Nr. 46 oder 48, kodiert wird, inhibiert, welches die Schritte aufweist:
a) die Verbindung mit einem System in Kontakt zu bringen, welches die *in vitro-*Bestimmung der Bindung zwischen TSAP6 und einem der Proteine, welches aus SEQ ID Nr. 1 bis SEQ ID Nr. 35 oder SEQ ID Nr. 44, 45 oder 47 ausgewählt oder durch eine Nukleinsäure, ausgewählt aus SEQ ID Nr. 36 bis SEQ ID Nr. 43 oder SEQ ID Nr. 46 oder 48, kodiert wird, erlaubt;
b) die Verringerung und/oder die Inhibition der Bindung zwischen TSAP6 und dem unter a) definierten Protein zu identifizieren.

2. Verfahren zur Identifizierung einer Verbindung, welche die Erhöhung der Tumorreversion und/oder des Zelltods (Apoptose) erlaubt, **dadurch gekennzeichnet, dass** es die Schritte aufweist:
a) Verbindungen mit einem System in Kontakt zu bringen, welches die in *vitro*-Bestimmung der Bindung zwischen TSAP6 und einem der Proteine, welches aus SEQ ID Nr. 1 bis SEQ ID Nr. 35 oder SEQ ID Nr. 44, 45 oder 47 ausgewählt oder durch eine Nukleinsäure, ausgewählt aus SEQ ID Nr. 36 bis SEQ ID Nr. 43 oder SEQ ID Nr. 46 oder 48, kodiert wird, erlaubt;
b) die Verbindungen zu identifizieren, welche die Verringerung und/oder die Inhibition der Bindung zwischen TSAP6 und dem unter a) definierten Protein induzieren;
c) die in Schritt b) identifizierten Verbindungen in einem *in vitro*-System in Kontakt zu bringen, welches erlaubt, die Phänomene von Apoptose und/oder von Tumorreversion zu messen;
d) die Erhöhung der Tumorreversion und/oder des Zelltods (Apoptose) in dem Modell bezogen auf ein Vergleichsmodell, mit welchem die Verbindung nicht in Kontakt gebracht worden ist, zu identifizieren.

3. Verfahren zur Identifizierung einer Verbindung für die Verringerung und/oder die Inhibition der Tumorreversion und/oder des Zelltods (Apoptose), welches die Schritte aufweist:
a) Verbindungen mit einem System in Kontakt zu bringen, welches die *in vitro*-Bestimmung der Bindung zwischen TSAP6 und einem der Proteine, welches aus SEQ ID Nr. 1 bis SEQ ID Nr. 35 oder SEQ ID Nr. 44, 45 oder 47 ausgewählt oder durch eine Nukleinsäure, ausgewählt aus SEQ ID Nr. 36 bis SEQ ID Nr. 43 oder SEQ ID Nr. 46 oder 48, kodiert wird, erlaubt;
b) die Verbindungen zu identifizieren, welche die Verringerung und/oder die Inhibition der Bindung zwischen TSAP6 und dem unter a) definierten Protein induzieren;
c) die in Schritt b) identifizierten Verbindungen in einem *in vitro*-System in Kontakt zu bringen, welches erlaubt, die Phänomene von Apoptose und/oder von Tumorreversion zu messen;
d) die Verringerung und/oder die Inhibition der Tumorreversion und/oder des Zelltods (Apoptose) in dem Modell bezogen auf ein Vergleichsmodell, mit welchem die Verbindung nicht in Kontakt gebracht worden ist, zu identifizieren.

4. Verfahren zur Identifizierung der Regionen von TSAP6, welche beteiligt sind an der Bindung an eines der Proteine, welches aus SEQ ID Nr. 1 bis SEQ ID Nr. 35 oder SEQ ID Nr. 44, 45 oder 47 ausgewählt oder durch eine Nukleinsäure, ausgewählt aus SEQ ID Nr. 36 bis SEQ ID Nr. 43 oder SEQ ID Nr. 46 oder 48, kodiert wird, welches die Schritte umfasst:
a) Peptide, welche von dem Protein TSAP6 stammen, in einem System in Kontakt zu bringen, welches die *in vitro*-Bestimmung der Bindung zwischen TSAP6 und dem Protein, welches aus SEQ ID Nr. 1 bis SEQ ID Nr. 35 oder SEQ ID Nr. 44, 45 oder 47 ausgewählt oder durch eine Nukleinsäure, ausgewählt aus SEQ ID Nr. 36 bis SEQ ID Nr. 43 oder SEQ ID Nr. 46 oder 48, kodiert wird, erlaubt;
b) die Peptide zu identifizieren, welche die Verringerung der Bindung zwischen TSAP6 und dem Protein, welches aus SEQ ID Nr. 1 bis SEQ ID Nr. 35 oder SEQ ID Nr. 44, 45 oder 47 ausgewählt oder durch eine Nukleinsäure, ausgewählt aus SEQ ID Nr. 36 bis SEQ ID Nr. 43 oder SEQ ID Nr. 46 oder 48, kodiert wird, in dem System zur Folge haben.

5. Verfahren zur Identifizierung eines Produkts, welches eine Aktivität einer Erhöhung der Tumorreversion und/oder der Apoptose aufweist, **dadurch gekennzeichnet, dass** es die Schritte umfasst:
a) ein Verfahren nach einem der Ansprüche 1 oder 2 auszuführen,
b) das in Schritt a) selektierte Produkt insbesondere durch Aufpfropfen von Resten auf das chemische Grundgerüst zu modifizieren,
c) das in Schritt b) modifizierte Produkt in *in vitro*-Verfahren an geeigneten Modellen von Tumorreversion und/oder Apoptose zu testen,
d) das Produkt zu identifizieren, welches erlaubt, eine Tumorreversions- und/oder Apoptoseaktivität, welche höher ist als die Aktivität, die für das in Schritt a) selektierte Produkt erhalten wird, zu erhalten.

6. Verfahren zur Identifizierung eines Produkts, welches eine Aktivität einer Verringerung und/oder einer inhibition der Tumorreversion und/oder der Apoptose aufweist, **dadurch gekennzeichnet, dass** es die Schritte umfasst:
a) ein Verfahren nach einem der Ansprüche 1 oder 3 auszuführen,
b) das in Schritt a) selektierte Produkt insbesondere durch Aufpfropfen von Resten auf das chemische Grundgerüst zu modifizieren,
c) das in Schritt b) modifizierte Produkt in *in vitro*-Verfahren an geeigneten Modellen von Tumorreversion und/oder Apoptose zu testen,
d) das Produkt zu identifizieren, welches erlaubt, eine Tumorreversions- und/oder Apoptoseaktivität, welche bezogen auf die Aktivität, die für das in Schritt a) selektierte Produkt erhalten wird, verringert ist, zu erhalten.

7. Komplex, welcher gebildet wird aus einem TSAP6-Protein und einem der Proteine, welches aus SEQ ID Nr. 1 bis SEQ ID Nr. 35 oder SEQ ID Nr. 44, 45 oder 47 ausgewählt oder durch eine Nukleinsäure, ausgewählt aus SEQ ID Nr. 36 bis SEQ ID Nr. 43 oder SEQ ID Nr. 46 oder 48, kodiert wird.
